# EUROPEAN PATENT APPLICATION

(11) **EP 2 974 741 A1**
(43) Date of publication of application: **20.01.2016**
(21) Application number: 14306156.2
(22) Date of filing: 16.07.2014
(51) Int. Cl.: A61K 39/36

(54) **Composition of allergen extracts having reduced toxicity and method of production thereof**

(71) Applicant: STALLERGENES, 92160 Antony (FR)
(72) Inventor: The designation of the inventor has not yet been filed
(74) Representative: Blot, Philippe Robert Emile

(57) **Abstract**

The present invention concerns allergen extracts, in particular ragweed *(Ambrosia)* pollen allergen extracts, containing reduced amounts of copper-binding-like proteins in order to minimize the risk of toxicity, including induction of angioedema, of those allergen extracts. The invention also relates to methods of preparing allergen extracts containing reduced amounts of copper-binding-like proteins.

## Description

The present invention concerns allergen extracts, in particular ragweed *(Ambrosia)* pollen allergen extracts, containing reduced amounts of copper-binding-like proteins in order to minimize the risk of toxicity, including induction of angioedema, of those allergen extracts. The invention also relates to methods of preparing allergen extracts containing reduced amounts of copper-binding-like proteins.

Weeds, and in particular weeds belonging to the genera *Ambrosia,* are often associated with pollinosis in the population exposed thereto. For instance, pollen of short ragweed *(Ambrosia artemisiifolia*) is clinically the most important source of seasonal aeroallergens in North America, as it is responsible for the majority and most severe cases of hay fever (allergic rhinitis). Because of the increased spreading of this plant, as well as the increased duration of pollination, there has been considerably more human exposure to short ragweed pollen and thus, the reported cases of hay fever due to short ragweed have risen dramatically.

Allergen immunotherapy (AIT), or desensitization, is a form of immunotherapy for allergic disorders in which the patient is administered an allergen preparation with the aim of inducing regulatory immune response. The sublingual route has been recently explored in the field of AIT. Allergen-specific sublingual immunotherapy (SLIT) indeed represents a safe and efficient non invasive alternative to subcutaneous immunotherapy (SCIT).

During development of a solid formulation of ragweed pollen extract, the Applicant was faced with a major adverse event during a phase I clinical trial, with occurrence of angioedema upon administration of the formulation in about 10% of the patients undergoing the clinical trial. These adverse events were not typical IgE-dependent allergic reactions, since: (1) they appeared remote from the administration site ; (2) they did not resolve after administration of antihistamine and corticoids; (3) their onset and (4) their duration were longer, and sometimes much longer, than expected from a typical IgE-dependent allergic reaction. The inventors concluded that they were due to Kinin-mediated vascular permeability.

A mouse model mimicking Kinin-mediated enhanced vascular permeability was developed and used to identify compounds responsible for the angioedema. Copper binding-like proteins were identified as being responsible for the induction of vascular permeability.

Accordingly, methods of preparing allergen extracts containing reduced amounts of copper-binding-like proteins have been developed which make it possible to provide allergen extracts containing reduced amounts of copper-binding-like proteins hence presenting reduced risk of angioedema.

### Methods of preparing allergen extracts

The invention thus relates to a method of preparing a purified allergen extract, which method comprises the step consisting of reducing the amount of copper binding-like proteins in an allergen extract.

In particular, the method of preparing a purified allergen extract comprises the steps consisting of:
a) preparing or providing an allergen extract; and
b) reducing the amount of copper binding-like proteins in said allergen extract.

By "providing an allergen extract", it is intended that an allergen extract which has been prepared beforehand, and usually stored, is obtained and used in the frame of the invention.

The method may also include the step of preparing the allergen extract.

An allergen extract is typically prepared by extraction of allergen(s) from a biological source material. The biological source material is typically a multicellular or non-cellular material from a multicellular organism of the Fungi, Plantae or Animal kingdom. Non limitative examples of allergen extracts include pollen extract (such as tree, herb, weed, and grass pollen), insect extracts (such as saliva and venom allergens, e.g., cockroach and midges, hymenopthera venom), mite and animal extracts (e.g. dog, cat, horse, rat, mouse), and food extracts (e.g. peanut, egg, milk). Important pollen allergens extracts are such allergen extracts of the genus *Ambrosia;* allergen extracts of the genus *Lolium;* allergen extracts of the genus *Cryptomeria*; allergen extracts of the genus *Alternaria*; allergen extracts of the genus *Alder,* allergen extracts of the genus *Betula;* allergen extracts of the genus *Quercus;* allergen extracts of the genus *Olea*; allergen extracts of the genus *Artemisia;* allergen extracts of the genus *Plantago*; allergen extracts of the genus *Parietaria;* allergen extracts of the genus *Cupressus;* allergen extracts of the genus *Thuya;* allergen extracts of the genus *Chamaecyparis;* allergen extracts of the genus *Periplaneta*; allergen extracts of the genus *Agropyron*; allergen extracts of the genus *Secale*; allergen extracts of the genus *Triticum*; allergen extracts of the genus *Cynorhodon*; allergen extracts of the genus *Juniperis;* allergen extracts of the genus *Dactylis*; allergen extracts of the genus *Festuca*; allergen extracts of the genus Poa; allergen extracts of the genus *Avena*; allergen extracts of the genus *Holcus;* allergen extracts of the genus *Anthoxanthum*; allergen extracts of the genus *Arrhenatherum;* allergen extracts of the genus *Agrostis*; allergen extracts of the genus *Phleum*; allergen extracts of the genus *Phalaris;* allergen extracts of the genus *Paspalum*; and allergen extracts of the genus *Sorghum.*

Said biological source is preferably a material from a non-genetically engineered organism. More particularly, purified recombinant allergens from cells genetically engineered for producing said allergens *in vitro* are excluded from the scope of the invention.

The allergen extract may comprise or consist of: a raw allergen extract; a concentrated allergen extract; or several allergens purified from an allergen extract. The allergens are naturally occurring allergens. An allergenic extract may naturally contain one or more isoforms of the same allergen. The allergen extract can also consist of a mixture of at least two allergen extracts of different biological sources.

According to an embodiment, the allergen extract comprises or consists of a ragweed (i.e. a plant of the genus *Ambrosia)* pollen allergen extract, in particular "short ragweed" *(Ambrosia artemisiifolia*) pollen allergen extract.

*Ambrosia artemisiifolia* pollen notably contains the allergens Amb a 1 and Amb a 11, which are major allergens, as well as Amb a 3, Amb a 4, Amb a 5, Amb a 6, Amb a 7, Amb a 8, Amb a 9, and Amb a 10, the other allergens identified so far. A reference sequence of Amb a 1 is available in the database GenBank under accession number AAA32665 (version AAA32665.1) and was described in Rafnar et al., J. Biol. Chem. 1991; 266: 1229-1236 (Amb a 1.01) together with its natural isoforms Amb a 1.02 and Amb a 1.03. Griffith et al., Int. Arch. Allergy Appl. Immunol. 1991; 96: 296-304 described another natural isoform of Amb a 1 (Amb a 1.04). At last, a reference sequence of Amb a 1.05, initially referred as Amb a 2, is available in the database GenBank under the accession number AAA32671 (version AAA32671.1) and was described in Rogers et al., J. Immunol. 1991; 147:2547-2552. Likewise, a reference sequence of Amb a 11 is available in the database GenBank under accession number KF528831 (version KF528831.1) and was described in the Patent international application PCT/EP2013/066358 (publication reference: WO2014/020179).

An allergen extract is conventionally prepared by extracting allergens from their biological source (e.g. pollen), which may consist in one or several sources (e.g. different species), with an aqueous solution, followed by separation, and then clarification.

Non limitative examples of aqueous solutions include water, purified water, ammonium bicarbonate, phosphate buffered saline (hereinafter PBS), Na phosphate, Na citrate, Na acetate, Na succinate, Na carbonate, Glycine HCL, Tris, preferably at a pH between 7.0 and 8.5. The extraction step is carried out by contacting the aqueous solution and the allergen biological source material, to form an extraction mixture, under agitation and in conditions sufficient for extracting allergens from the biological source material, typically for 1h to 24h (preferably 20h to 24h) at 1 °C to 20 °C (preferably 2-9 °C), under for instance conditions of mechanical stirring.

The separation step enables separating solid residues of the biological source material from the liquid phase containing the extracted allergens, to recover the liquid phase. This separation can be performed, for instance, by centrifugation. This is then followed by a clarification step, for instance by one or more filtration(s) of the collected supernatant, or directly by filtration of the extraction mixture.

Optionally, the allergen extract may be further concentrated. This may be done for instance by ultrafiltration or diafiltration on a 1-10 kDa membrane, preferably 1-5 kDa membrane, still preferably with a 1 kDa membrane. Molecules having a molecular size of less than the cut-off value in kDa are thereby removed.

According to an embodiment, a diafiltration is performed with at least 2.5 volumes, preferably 5, 10, 11, 12, 13, 14 or 15 volumes, of an aqueous solution selected from the group consisting of purified water, bicarbonate solution or another buffered solution such as phosphate buffered solution (patent application PCT/EP2010/057935). Mannitol may be added, such as with a 2% final ratio (weight/weight). The method of preparing purified, partially purified or enriched allergen extract according to the invention may further comprise a step of filtration on a 0.22 µm filter.

At last, the allergen extract can further be freeze-dried and formulated into an appropriate pharmaceutical composition, e.g. a tablet.

The method according to the invention comprises the step of reducing the amount of copper binding-like proteins in the allergen extract.

As used herein, the term "copper binding-like protein(s)" (or "copper binding protein(s)") denotes proteins presenting a domain(s) that appear(s) suitable, as identified by structural homology with copper binding proteins, to bind one or more copper ion(s) (Cu⁺ or Cu²⁺). Copper atoms are predominantly ligated by histidine, cysteine, and methionine residues to form tetrahedral like complexes. Copper binding-like proteins are generally classified in two groups: those that utilise copper as a cofactor necessary for their biological function and/or those involved in copper trafficking proteins are those that transport copper as cargo.

Reducing the amount of copper binding-like proteins is intended to mean that the amount of at least one copper binding-like protein in the allergen extract is reduced. In some embodiments, the amount of at least two, three, four or more copper binding-like proteins present in the allergen extract is reduced.

Copper binding-like proteins include in particular *Ambrosia artemisiifolia* proteins A, B, C and D as identified in the following examples.

Protein A is a 139 amino acid long protein, in its non-matured form, which sequence is shown in SEQ ID NO:2. A nucleotide sequence encoding the pro-protein A is shown in SEQ ID NO:1. The predicted mature form of protein A comprises amino acids at positions 25 to 139 of SEQ ID NO:2 (SEQ ID NO:3). Two naturally occurring variants of the polypeptide consisting of sequence SEQ ID NO:2 have been identified: Ile>Leu at position 15 of SEQ ID NO:2, and Gln>Gly at position 65 of SEQ ID NO:2.

Protein B is a 122 amino acid long protein, in its non-matured form, which sequence is shown in SEQ ID NO:5. A nucleotide sequence encoding the pro-protein B is shown in SEQ ID NO:4. The predicted mature form of protein B comprises amino acids at positions 25 to 122 of SEQ ID NO:5 (SEQ ID NO:6).

Protein C is a 144 amino acid long protein, in its non-matured form, which sequence is shown in SEQ ID NO:8. A nucleotide sequence encoding the pro-protein C is shown in SEQ ID NO:7. The predicted mature form of protein C comprises amino acids at positions 25 to 144 of SEQ ID NO:5 (SEQ ID NO:9).

Protein D is a 122 amino acid long protein, in its non-matured form, which sequence is shown in SEQ ID NO:11. A nucleotide sequence encoding the pro-protein D is shown in SEQ ID NO:10. The predicted mature form of protein D comprises amino acids at positions 25 to 122 of SEQ ID NO:5 (SEQ ID NO:12). Preferably, the amount of at least one copper binding-like protein which has at least 30%, 40%, 45% or 50% sequence identity with the protein A consisting of sequence SEQ ID NO:3 is reduced. Still preferably, the amount of at least one copper binding-like protein which has at least 30%, 35%, 38% or 40% sequence identity with the protein A consisting of sequence SEQ ID NO:2 is reduced.

A protein having an amino acid sequence, for example, at least 95% "identical" to sequence SEQ ID NO: 3 is a protein which amino acid sequence, after global alignment with SEQ ID NO: 3, includes no more than five amino acid modifications per each 100 amino acids of the sequence SEQ ID NO: 3 in the alignment (including gaps). In other words, to obtain a polypeptide having an amino acid sequence at least 95% identical to the sequence SEQ ID NO: 3, up to 5% (5 of 100) of the amino acid residues in the subject sequence may be inserted, deleted, or substituted with another amino acid.

The percentage of identity between two sequences determined by global pairwise alignment may be calculated using the Needleman-Wunsch algorithm, as implemented for instance by the program EMBOSS Needle (protein), with the EBLOSUM62 matrix, and the following parameters: gap-open=10, gap-extend=0.5, endopen 10.0, endextend 0.5.

The percentages of identity of the protein A with, respectively, protein B, protein C and protein D as calculated with EMBOSS Needle (using the same parameters as listed above) are shown in the Table 5.

Proteins B, C and D make part of the copper binding-like proteins which amount is reduced is some embodiments of the invention.

According to an embodiment, said at least one copper binding-like protein which amount is reduced comprise(s) (i) a protein consisting of SEQ ID NO. 2, a fragment of SEQ ID NO. 2 comprising SEQ ID NO:3, or a protein consisting of SEQ ID NO.3; and/or a protein consisting of SEQ ID NO. 5, a fragment of SEQ ID NO. 5 comprising SEQ ID NO:6, or a protein consisting of SEQ ID NO. 6.

The step consisting of reducing the amount of copper binding-like proteins in the extract can be performed by filtration, size exclusion, ion-exchange, hydrophobic interaction, affinity, in particular metal chelate, antibody and aptamer affinity, or mixed mode chromatography, or phase separation.

The actual reduction of the amount of copper binding-like proteins can be assayed:
- directly, by measuring, as non-limiting examples, the amount of copper binding proteins by mass spectrometry (MS) using a combination of reversed-phase liquid chromatography. For instance, in a label-free quantitation of the copper binding proteins (CuBPs), same protein amounts from each fraction are first denaturated using ProteaseMAX surfactant (Promega) at 0.025% and reduced with 5 mM dithiothreitol (DTT, Sigma) at 56 °C for 20 min. Next, cysteines are alkylated with 10 mM of iodoacetamide at room temperature for 30 min and then proteins are digested with trypsin at 37 °C for 3 h. The tryptic digestion is stopped by adding 1% of formic acid. Degraded surfactant is removed by centrifugation at 14000 rpm for 5 min. The protein digest is then subjected to nanoRP-HPLC-UV-ESI-MS and MS/MS analyses using a Maxis 4G mass spectrometer (Bruker Daltonics) coupled with an ultra high performance liquid chromatography (nanoRSLC Ultimate 3000, Dionex). Tryptic digests is first desalted in-line using an Acclaim PepMap100 nanoViper pre-column (C18, 2 µm, 100 A, 100 µm i.d x 2 cm; Thermo) under loading buffer (2% ACN; 0.15 % formic acid in water) for 10 minutes at 35 °C and eluted to a Acclaim PepMapRSLC nanoViper column (C18, 2 µm, 100 A, 75 µm i.d. x 25 cm; Thermo), beforehand equilibrated with 99 % solvent A (A: 0.15 % formic acid in water) and 1 % solvent B (B: 80 % ACN; 0.15 % formic acid in water). Peptides are eluted using the following gradient: 0-10 min, 1 % B; 10-35 min, 1-42 % B; 35-39 min, 42-60 % B and 39-40 min, 60-95 % B at a flow rate of 450 nL/min. Mass spectra are acquired in the mass range of 100-2000 m/z and MS/MS experiments are performed on the two most abundant ions for each MS scan. Singly charged ions are not subjected to MS/MS and ions already fragmented are dynamically excluded for 10 seconds. Three independent LC-MS runs are performed for each fraction. LC-MS Progenesis software (Non-Linear Dynamics, Waters Company) is used for the relative quantitation of CuBPs. Briefly, LC-MS analyses acquired in triplicate for each fraction are loaded in LC-MS Progenesis and are aligned against a reference run automatically selected by the software. Peptide features are identified by importing the Mascot search results. Protein abundances are calculated (without normalization) from the sum of the precursor intensities of all peptides leading to the identifications of copper binding-like proteins. Conflicting peptides features are excluded from the quantitation.

The method to identify copper binding proteins may also be based on immunological methods (western blotting or ELISA) using either polyclonal or monoclonal antibodies or a combination of both.
- or indirectly, using a model of kinin-mediated vascular permeability or an *in vitro* HUVECs (Human umbilical vein endothelial cells) monolayer permeability assay. For instance, this can be made by measuring the amount of Evans blue dye in footpads of a mouse model of vascular permeability as described in the following Example 2. Briefly, mice are sensitized with 2 intra-peritoneal (i.p.) injections of allergen extract (e.g. using doses equivalent to 10 µg of major allergen) on days 0 and 14 in presence of 2 mg aluminum hydroxide, then exposed from days 21 to 24 to an aerosolized extract of the same allergen(s) 20 min per day, using similar doses as above. Preferably, on day 25, dorsal hairs are removed. On day 28, mice are anesthetized and then receive : (i) an intradermal injection of mannitol (e.g. 50 µL, 46 mg/mL in PBS) on the left dorsal flank and (ii) on the right dorsal flank: (iii) for part of the mice: either a natural purified major allergen present in the allergen extract to be assayed (e.g. 50 µL, dose 25 µg) or mannitol (e.g. 50 µL, 46 mg/mL in PBS) as negative controls, or (iv) the allergen extract in which the amount of copper binding-like proteins is to be reduced (e.g. 50 µL, dose containing 25 µg major allergen) as a positive control, or (v) the test product (purified allergen extract e.g. 50 µL, adjusted to 25 µg of the purified major allergen). Immediately after injection, Evans blue (100 µL, 1% in PBS) is administered by retro-orbital injection. After 35 min, the mice are euthanized and skin (2 injection sites; left and right dorsal flanks) and feet are removed and placed into dimethylsulfoxide (e.g. 0.5 mL and 1 mL, respectively). The Evans blue dye is extracted from the skin and feet and measured by spectrophotometry at 620 nm using a standard range of Evans blue. Comparisons of the concentrations of extravasated Evans blue in footpads are used to determine if the amount of copper binding-like proteins has been reduced in the test products (allergen extract purified according to the method of the invention).

Alternatively, this can be assayed using an *in vitro* HUVECs monolayer permeability assay *(See for example* Sen et al., "Homocysteine induced myofibroblast differentiation in mouse aortic endothelial cells", 2006, J Cell Physiol 209: 767-774). Briefly, HUVECs are cultured in Transwell chambers until reaching confluency. FITC-Dextran is added in the upper chamber, allowing it to permeate through the cell monolayer. Permeability can be determined by measuring the fluorescence of the plate well solution in the lower chamber.

In an embodiment, the step consisting of reducing the amount of copper binding-like proteins in the allergen extract is performed by ultrafiltration. Ultrafiltration of the allergen extract in aqueous solution can be performed on a 10-70 kDa membrane, in particular a 13-37 kDa, and more particularly 15-30 kDa.

Preferably, a surfactant is added to the allergen extract before ultrafiltration is performed. According to an embodiment, the surfactant is selected from the group consisting of 3-[(3-cholamidopropyl)dimethylammonio]-1-propanesulfonate (CHAPS), octylβglucosides (preferably Octyl β-D-glucopyranoside), Polyoxyethylenesorbitan monooleate (Tween@ 80), polyoxyethylenesorbitan monolaurate (Tween@ 20), and combinations thereof. The concentration of surfactant may range from 0.001 to 5% (w/w), preferably from 0.01 to 1% (w/w). It is preferred that the concentration does not exceed the critical micellar concentration.

Advantageously, the mixture of allergen extract and surfactant is incubated before ultrafiltration. Incubation is performed for instance for 1 to 60 min, e.g. from 10 to 40 min, in particular for 20 to 30 min, at room temperature or from 1 to 22 °C.

Accordingly, in an embodiment the method according to the invention comprises the steps consisting of:
a) adding a surfactant into an allergen extract in aqueous solution;
b) incubating the mixture of allergen extract and surfactant;
c) ultrafiltrating the mixture on a 10-70 kDa membrane;
d) collecting the retentate.

Where the method also includes the steps of preparing the allergen extract, said method comprises the steps consisting of:
i) contacting an aqueous solution and an allergen biological source material, to form an extraction mixture, under agitation and in conditions sufficient for extracting allergens from the biological source material,;
ii) separating solid residues from the liquid phase containing the extracted allergens in aqueous solution;
iii) collecting the liquid phase which consists of the allergen extract;
iv) clarifying the allergen extract;
v) optionally concentrating the allergen extract;
vi) optionally mixing the allergen extract (concentrated if step (v)) was performed or non-concentrated if step (v)) was not performed) with at least one allergen extract (concentrated or non-concentrated) from another biological source; and
   a) adding a surfactant into the allergen extract obtained at step iii), iv) or v);
   b) incubating the mixture of allergen extract and surfactant;
   c) ultrafiltrating the mixture on a 10-70 kDa membrane;
   d) collecting the retentate.

As used herein, "optionally" means that the step is either performed or not performed in the course of implementation of the method according to the invention.

The collected retentate consists of the allergen extract in which the amount of copper binding-like proteins has been reduced.

Preferably, the step of ultrafiltration on 10-70 kDa membrane is performed in conditions such that the allergen content of the allergen extract is reduced by less than 60% of its initial content, preferably less than 50%, preferably less than 40 %, preferably less than 35%.

The ultrafiltration retentate is further treated in order to remove the surfactant. This can be conventionally made by dialysis or diafiltration with a 10 kDa membrane. Advantageously, dialysis or diafiltration is performed using a pharmaceutically acceptable solvent such as PBS. In the case of dialysis, the filtrate is further concentrated. Dialfiltration provides for a retentate which is already concentrated.

According to another embodiment, reducing the amount of copper binding-like proteins in the extract is performed by affinity chromatography, using antibodies directed against copper binding-like proteins.

For instance, in order to reduce the amount of copper binding-like proteins in ragweed (and in particular short ragweed) pollen extract, affinity chromatography with antibodies specifically directed against protein A (mature form) is performed. Preferably a second step of affinity chromatography with antibodies specifically directed against protein B (mature form) is also performed. Additional steps with affinity chromatography with antibodies specifically directed against protein C and/or protein D (mature forms), or another variant of Protein A, B, C or D is also perfomed. Alternatively, affinity chromatography may be performed concomitantly with any combinations of antibodies directed respectively to any combinations of proteins A, B, C and D, and their respective variants. Advantageously, the collected fractions are further submitted to dialysis or a pharmaceutically acceptable solvent such as PBS.

Antibodies specifically directed against protein A, B, C or D are readily available to the skilled person, based on the known sequences of these proteins and the methods conventionally used in the art for producing monoclonal or polyclonal antibodies.

According to a further embodiment, reducing the amount of copper binding-like proteins in the extract is performed using aptamers directed against copper binding-like proteins.

For instance, in order to reduce the amount of copper binding-like proteins in ragweed (and in particular short ragweed) pollen extract, aptamers specifically directed against protein A (mature form) are used. Preferably a second step with aptamers specifically directed against protein B (mature form) is also performed. Additional steps with aptamers specifically directed against protein C and/or protein D (mature forms), or another variant of protein A, B, C or D is also performed.

Aptamers specifically directed against protein A, B, C or D are readily available to the skilled person, based on the known sequences of these proteins and the methods conventionally used in the art for producing aptamers.

For instance, the aqueous allergenic extract can be incubated with beads bearing the aptamers of interest and the beads to which toxic proteins are bound are physically removed by filtration of centrifugation, therefore yielding a copper binding-like protein depleted allergenic extract.

A similar approach can be performed using chromatography, where the eluted extract is depleted from copper binding-like protein.

Finally, the aptamer-based affinity media can be sanitized and regenerated by incubation in selection buffer and stored until future use.

The invention also relates to a purified allergen extract which has been obtained, or is obtainable, by the method according to the invention.

Optionally the purified allergen extract and another purified allergen extract (from another biological source, for instance) are combined before being formulated into a pharmaceutical composition.

Advantageously, the method of preparing a purified allergen extract further comprises the step of formulating said purified allergen extract into a pharmaceutical composition.

A pharmaceutical composition typically comprises an active principle (the purified allergen extract) and a pharmaceutically acceptable excipient.

Accordingly, formulating the purified allergen extract into a pharmaceutical composition may consist in changing the solvent in which the allergen extract is present after its purification, if this solvent is not pharmaceutically acceptable. This can conventionally be made by dialysis or diafiltration, for instance.

The purified allergen extract can also be dried, e.g. by freeze-drying or spray-drying, for subsequent storing or for formulation into a solid pharmaceutical composition. For freeze-drying a cryoprotectant, such as mannitol, is conventionally added to the purified allergen extract.

Pharmaceutical compositions obtained, or obtainable, by the method according to the invention are described in more details in the following section.

### Pharmaceutical compositions and therapeutic indications

The invention further relates to a pharmaceutical composition which is obtained, or obtainable, by the method of the invention.

As used herein, the term "pharmaceutically acceptable excipient" includes solvents, dispersion media, coatings, antibacterial and antifungal agents, isotonic and absorption delaying agents, mucoadhesive excipients, and the like, that do not produce an adverse or other untoward reaction when administered to an animal, or a human, as appropriate. Excipients may further include, but are not limited to, disintegrants, binders, lubricants, flavoring, colorants, preservatives.

The pharmaceutical compositions can further include any conventional adjuvant, such as aluminium hydroxide or calcium phosphate, or delivery vector used in allergen specific immunotherapy.

The pharmaceutical composition according to the invention can be administered in forms suitable for mucosal, subcutaneous, epicutaneous, intradermal, intralymphatic or intraveinous routes. Preferably, the pharmaceutical composition is to be administered by the mucosal route, more preferably by the oromucosal route, and most preferably by the perlingual or sublingual route. As such the pharmaceutical composition and the medicament are preferably formulated in a way adapted for such administration routes.

Mucosal administration denotes any administration method, wherein the formulation in part or in full comes into contact with a mucosa. Mucosa refers to the epithelial tissue that lines the internal cavities of the body. The mucosal surface is preferably selected from the group consisting of a nasal, buccal, oral, gastrointestinal and pulmonary tract.

Oromucosal administration comprises any administration method, wherein the formulation in part or in full comes into contact with the mucosa of the oral cavity and/or the pharynx of the patient. It includes in particular sublingual, perlingual (i.e. through the tongue mucosa) and oral administrations.

The pharmaceutical composition according to the invention can be administered in various forms, such as dispersed forms, e.g. in solutions, suspensions (liquids) or gels, sprays, or as solid dosage forms.

"Solid dosage form" means the dosage form of the allergen is solid form, for example capsules, tablets, pills, powders, lyocs, dragees or granules. In such solid dosage forms, the allergen is admixed with at least one inert customary excipient (or carrier) such as sodium citrate or dicalcium phosphate or (a) fillers or extenders, as for example, starches, trehalose, lactose, sucrose, glucose, mannitol and silicic acid, (b) binders, as for example, carboxymethylcellulose, alignates, gelatine, polyvinylpyrrolidone, sucrose and acacia, (c) humectants, as for example, glycerol, (d) disintegrating agents, as for example, agar-agar, calcium carbonate, potato or tapioca starch, alginic acid, certain complex silicates and sodium carbonate, (e) absorption accelerators, as for example, quaternary ammonium compounds, (f) wetting agents, as for example, cetyl alcohol and glycerol monostearate, (g) adsorbents, as for example, kaolin and bentonite, (h) lubricants, as for example, talc, calcium stearate, magnesium stearate, solid polyethylene glycols, sodium lauryl sulfate, (i) opacifying agents, and (j) buffering agents.

The solid pharmaceutical composition may be compressed or non-compressed solid dosage form, in particular compressed or non-compressed tablet.

A "non-compressed" solid dosage form denotes a solid dosage form which is manufactured by removal of a liquid from a solidified system comprising matrix forming agents, active ingredient and other suitable ingredients resulting in active ingredient comprised solid matrix.

Preferably, the solid pharmaceutical composition is formulated in the form of a tablet for sublingual or perlingual administration.

Preferably, the solid pharmaceutical composition is a fast-dispersing composition, in particular a fast-dispersing tablet.

In particular, the solid dosage form may be a "lyoc", i.e. a lyophilised solid dosage form for fast dispersion in the mouth and/or underneath the tongue. Such a solid dosage formulation may for instance typical contain gelatine, mannitol and sodium hydroxide as excipients.

The pharmaceutical composition preferably comprises a dose of allergen(s) effective for desensitization, i.e. a dose which taken once or repeatedly in the frame of allergen specific immunotherapy enables to desensitize a patient allergic to allergen(s) present in the pharmaceutical composition.

The amount of relevant allergen can be determined for instance by means of immunoassays (such as ELISA, radial immunodiffusion (RID) and rocket immunoelectrophoresis (RIE)) or by mass spectrometry.

The solid pharmaceutical composition may comprise for instance a total amount of 0.01-10,000 µg, preferably 0.01-1000 µg, preferably 0.1-500 µg of allergens, more particularly of major allergens, more particularly of Amb a 1 and/or Amb a 11.

The allergen content of the composition may be expressed in term of biopotency (allergenic activity as measured in vivo and/or in vitro) for which there is currently no internationally accepted standardised method. The bio-potency of a given extract mostly depends on the content of relevant allergens in the extract, which content varies with the biological source material. Different units have been developed such as IR (index of reactivity), BAU (Bioequivalent Allergen Units), AU (Allergy Units), SQ-Units (Standardised Quality Units). Index of Reactivity or IR are established on the basis of Stallergenes' biopotency IR standardised method. The IR can be determined by means of an immunoassay such as inhibition ELISA assay. 100 IR containing grass pollen extracts equal around 3000 BAU. BAU or Bioequivalent Allergen Units is the biopotency units established on the basis of the FDA requirements described in "Methods of the Allergenics Products Testing Laboratory", October 1993, Docket No. 94N-0012 at p. 15.

As to its potency, the solid pharmaceutical composition may comprise for instance a total value of 0.01-10,000 IR, preferably 0.1-1,000 IR, preferably 1-500 IR, preferably 100-300 IR.

The invention relates in particular to a solid pharmaceutical composition containing a purified ragweed pollen extract, in particular short ragweed pollen extract, in which copper binding-like proteins are in amounts such that they do not induce vascular permeability. By vascular permeability it is meant the permeability of blood and/or lymphatic vessels.

The induction (or lack of induction) of vascular permeability can be determined using a model of kinin-mediated vascular permeability model, in particular a mouse model of vascular permeability as described above or any other suitable method such as the HUVECs assay also previously described.

More specifically, the solid pharmaceutical composition contains copper binding-like proteins in amounts such that they do not induce vascular permeability if the solid pharmaceutical composition, when administered to the model, does not induce higher vascular permeability compared to a control (negative control, such as a pharmaceutically acceptable solvent).

The invention also concerns a composition according to the invention for use for allergy treatment and/or prevention (desensitization). Advantageously, due to the reduction of the amount of copper binding-like proteins in the pharmaceutical composition, said allergy treatment and/or prevention is associated with reduced risk of vascular permeability.

The invention also relates to a method for allergy desensitization, which method comprises administering a patient in need thereof with a pharmaceutical composition according to the invention.

In the context of the invention, the terms "desensitization" means preventing, reversing, alleviating, or inhibiting the course of a pathological reaction of the immune system leading to the manifestation of allergy, or one or more symptoms thereof. The treatment can be applied to a subject that is not sensitised to ragweed, more particularly short ragweed, that is sensitised to ragweed, more particularly short ragweed, but has not yet experienced allergic symptoms associated with said allergen, as well as subjects that are sensitised to said allergen and are experiencing symptoms of allergy. The treatment can also be applied in relation to any other allergen that shows cross-reactivity with ragweed, more particularly short-ragweed, allergens.

Symptoms of allergy typically include rhinitis, conjunctivitis, asthma, urticaria, eczema, or more generally reactions in the skin, eyes, nose, upper and lower airways with common symptoms such as redness and itching of eyes and nose, runny nose, and wheezing.

AIT or desensitization therapy is conventionally carried out by administering repeatedly a monodose or incremental doses of an allergen to a patient in need thereof, thereby resulting in an adaptive immune response of the patient who becomes desensitised to the allergen.

### Ambrosia artemisiifolia copper-like binding proteins

The invention also relates to isolated short ragweed copper binding proteins and homologs thereof, and to nucleic acid sequence comprising a polynucleotide sequence encoding these proteins.

An isolated protein comprising a sequence consisting of SEQ ID NO:3, SEQ ID NO:6, SEQ ID NO:9 or SEQ ID NO:12, or of a sequence at least 40%, 50%, 70%, 85%, 90% or 95% identical to any of SEQ ID NO:3, SEQ ID NO:6, SEQ ID NO:9 or SEQ ID NO:12 thus makes part of the invention.

Preferably, the invention is drawn to an isolated protein comprising sequence SEQ ID NO:2, SEQ ID NO:5, SEQ ID NO:8, and SEQ ID NO:11, or a sequence at least 40%, 50%, 70%, 85%, 90% or 95% identical to any of SEQ ID NO:2, SEQ ID NO:5, SEQ ID NO:8, and SEQ ID NO:11.

According to an embodiment said isolated protein is a copper binding-like protein. Additionally or alternatively, said protein induces kinin-mediated vascular permeability when administered to a Mammal, e.g a rodent, in particular a mice, in an amount sufficient for inducing said kinin-mediated vascular permeability. The capacity for inducing kinin-mediated vascular permeability can be readily assayed for instance by measuring the amount of Evans blue dye in footpads of a mouse model of vascular permeability, as described in the section *"Methods of preparing allergen extracts"* of the instant application and in example 2.

In an embodiment, the protein according to the invention is a fusion protein including a second sequence foreign or heterologous to the first sequence consisting of SEQ ID NO:3, SEQ ID NO:6, SEQ ID NO:9 or SEQ ID NO:12, or of a sequence at least 40% thereto. By "foreign" or "heterologous" it is meant that the second sequence is not a genomic sequence contiguous to the first sequence consisting of SEQ ID NO:3, SEQ ID NO:6, SEQ ID NO:9 or SEQ ID NO:12, or of a sequence at least 40% thereto. Thus preferably, said protein is artificial, i.e. not naturally found in nature. Said second foreign or heterologous sequence may be for instance a tag such as a His-Tag, or a thioredoxin (TRX) sequence. Examples of such sequences are shown in SEQ ID NO:24 and SEQ ID NO:26.

A protein as disclosed therein can be readily produced by means of recombinant DNA technology, by transforming a producing cell, such as a bacteria or yeast with the nucleic acid sequence encoding said protein. Reference is made for instance to Molecular Cloning: A Laboratory Manual (Fourth Edition) by Michael R. Green and Joseph Sambrook, Cold Spring Harbor Laboratory Press, for a description of methods available to the skilled person.

An isolated nucleic acid sequence encoding said protein is also part of the invention. Preferably said nucleic acid comprises a cDNA sequence encoding the protein of the invention.

The nucleic acids encoding proteins of sequence SEQ ID NO: 2-3 and 5-6 make part of the transcriptome of *Ambrosia artemisiifolia* which was described by Kanter et al., Plos one, April 2013 | Volume 8 | Issue 4 | e61518 and was submitted to the European Nucleotide Archive (ENA) with the project accession: PRJEB1470 (ERX206182/ERX206183). However, these sequences are within raw contigs that make part of a large un-annotated bank.

The invention also relates to a vector or recombinant DNA sequence comprising the nucleic acid according to the invention operatively linked to a regulatory sequence heterologous to the nucleic acid according to the invention.

Isolated antibodies specifically binding to these proteins also make part of the invention. The antibodies may be monoclonal or polyclonal.

The term "monoclonal antibody" or "mAb" as used herein refers to an antibody molecule of a single amino acid composition, that is directed against a specific antigen and which may be produced by a single clone of B cells or hybridoma. Monoclonal antibodies may also be recombinant, i.e. obtained by protein engineering. Recombinant antibodies may be produced in a mammalian cell line such as CHO, NSO, PERC6 or any other cell after transfection.

The term "polyclonal antibodies" refers to a combination of immunoglobulins directed against a specific antigen, each immunoglobulin possibly binding to a different epitope on the antigen. Polyclonal antibodies are generally produced by immunisation of a suitable mammal, such as a mouse, rabbit or goat.

The invention also relates to use of a protein as defined above for the preparation of a hybridoma which secretes a monoclonal antibody directed against said protein or of a polyclonal antibody.

The invention thus relates to a method for preparing a polyclonal antibody directed against a protein as defined above, which method comprises:
- immunising an animal, such as a mouse, sheep, rabbit or rat, with a protein (or peptides, DNA sequence encoding said protein or part of it) as defined above;
- recovering the serum of the immunised animal;
- selecting the antibodies present in the serum that bind to the protein used for immunisation.

A method for preparing a hybridoma which secretes a monoclonal antibody directed against a protein as defined above is also provided, wherein :
- samples of lymphocytes secreting immunoglobulins are taken from an animal, such as a mouse, rabbit or rat, immunised with a protein as defined above,
- the lymphocytes are then fused with myeloma cells, such as Sp2 myeloma cells (ATCC CRL-1581), in order to obtain a hybridoma.

The present invention also relates to a hybridoma obtainable by the method for preparing a hybridoma defined above.

Generally, the methodology used to obtain hybridomas and monoclonal antibodies may follow the conventional method of lymphocyte fusion and hybridoma culture described by Köhler & Milstein (1975) Nature 256:495-497. Other methods for preparing monoclonal antibodies are also known (e.g., Harlow et al, ed. 1988 "Antibodies: a laboratory manual").

Alternative methods to this conventional method also exist. Monoclonal antibodies can be produced, for example, by expressing a nucleic acid cloned from a hybridoma.

The invention also relates to a method of detecting or quantifying ragweed copper binding-like proteins in a ragweed pollen extract, and more particularly short ragweed copper binding-like proteins in a short ragweed pollen extract, which comprises the steps consisting of:
a) contacting a ragweed, more particularly a short ragweed, pollen extract with antibodies according to the invention;
b) detecting immune complexes formed between the antibodies and ligands present in the ragweed, more particularly short ragweed, pollen extract;
c) deducing from the complexes detected the presence, absence or quantity of ragweed, and more particularly short ragweed, copper binding-like proteins in the short ragweed pollen extract.

The invention also relates to a method of depleting a short ragweed pollen extract from ragweed, and more particularly short ragweed, copper binding-like proteins using antibodies directed against said proteins. In particular, said method comprises:
a) contacting a ragweed, more particularly a short ragweed, pollen extract with antibodies according to the invention; and
b) removing immune complexes formed between the antibodies and ligands present in the ragweed, more particularly short ragweed, pollen extract.

Detection or removal of the immune complexes formed between the antibodies and ligands present in the ragweed, more particularly short ragweed, pollen extract may be made according to any method available to the skilled person. This can be conventionally performed using secondary antibodies directed against the antibodies contacted with the ragweed, more particularly short ragweed, pollen extract (for instance using anti-IgG antibodies if the antibodies according to the invention are IgGs).

The invention also relates to aptamers that bind to these proteins.

Aptamers are single-stranded oligonucleotides composed of RNA or DNA generated by systematic evolution of ligands by exponential enrichment (SELEX), an iterative process during which consecutive rounds of in-vitro selection and amplification enable the isolation of specific sequences owing to their affinity for a given target. During SELEX, aptamers are isolated from combinational libraries of synthetic oligonucleotides (containing up to 10¹⁵ different randomly synthesized candidates) with regard to their affinity toward the toxic protein to be depleted from the allergenic extract (Tuerk, C., Gold, L., Science 1990, 249, 505-510; Robertson, D. L., Joyce, G. F. Nature 1990, 344, 467-468; Ellington, A. D., Szostak, J. W., Nature 1990, 346, 818-822).

Once the specific aptamer of interest is obtained based on SELEX, it is possible to fabricate an affinity solid media bearing said aptamer. In order to immobilize the aptamer on the media (e.g. chromatography beads, magnetic beads) the deoxyribonucleic acid is chemically modified with a chemical group (e.g. thiols, amines) which covalently immobilizes said aptamer to the media, as described for instance in EP2398818A1.

After immobilization of the aptamer on a solid media, the aptamer is folded into the correct three-dimensional structure by incubation in the buffer that was used during the SELEX process. In the folded state, the aptamer is able to bind the target copper binding-like protein while other proteins from the allergenic extract will not attach to the aptamer.

The invention also relates to a method of detecting or quantifying ragweed copper binding-like proteins in a ragweed pollen extract, and more particularly short ragweed copper binding-like proteins in a short ragweed pollen extract which comprises the steps consisting of:
a) contacting a ragweed, more particularly short ragweed, pollen extract with aptamers according to the invention;
b) detecting complexes formed between the aptamers and ligands present in the ragweed pollen, more particularly short-ragweed extract;
c) deducing from the complexes detected the presence, absence or quantity of ragweed, and more particularly short ragweed, copper binding-like proteins in the ragweed, more particularly short ragweed, pollen extract.

The invention also relates to a method of depleting a short ragweed pollen extract from ragweed, and more particularly short ragweed, copper binding-like proteins using aptamers directed against said proteins. In particular, said method comprises:
a) contacting a ragweed, more particularly a short ragweed, pollen extract with aptamers according to the invention; and
b) removing complexes formed between the aptamers and ligands present in the ragweed, more particularly short ragweed, pollen extract.

Detection or removal of the complexes formed between the aptamers and ligands present in the ragweed, more particularly short ragweed, pollen extract may be made according to any method available to the skilled person. For instance, the aqueous allergenic extract is incubated with beads bearing the aptamers of interest and the beads to which the copper binding-like proteins are bound are physically removed by filtration or centrifugation, therefore yielding a copper binding-like protein depleted allergenic extract. A similar approach can be performed using chromatography, where the eluted extract is depleted from copper binding-like proteins.

Throughout the instant application, the term "comprising" is to be interpreted as encompassing all specifically mentioned features as well optional, additional, unspecified ones. As used herein, the use of the term "comprising" also discloses the embodiment wherein no features other than the specifically mentioned features are present (*i.e.* "consisting of") as well as the embodiment wherein features other than the specifically mentioned feature are present provided that the essential characteristics of the composition are not materially affected by their presence (i.e. "consisting essentially of").

The invention will be further illustrated by the following figures and examples. Throughout the figures and examples, ragweed is intended for short ragweed *(Ambrosia artemisiifolia*).

### SEQUENCES

SEQ ID NO:1 is the nucleotide sequence, including signal sequence, encoding pro-protein A.
SEQ ID NO:2 is the sequence of pro-protein A encoded by SEQ ID NO:1.
SEQ ID NO:3 is the sequence of the predicted mature form of protein A.
SEQ ID NO:4 is the nucleotide sequence, including signal sequence, encoding pro-protein B.
SEQ ID NO:5 is the sequence of pro-protein B encoded by SEQ ID NO:4.
SEQ ID NO:6 is the sequence of the predicted mature form of protein B.
SEQ ID NO:7 is the nucleotide sequence, including signal sequence, encoding pro-protein C.
SEQ ID NO:8 is the sequence of pro-protein C encoded by SEQ ID NO:7.
SEQ ID NO:9 is the sequence of the predicted mature form of protein C.
SEQ ID NO:10 is the nucleotide sequence, including signal sequence, encoding pro-protein D.
SEQ ID NO:11 is the sequence of pro-protein D encoded by SEQ ID NO:10.
SEQ ID NO:12 is the sequence of the predicted mature form of protein D.
SEQ ID NO:13 to SEQ ID NO:22 are sequences of peptides derived from protein A or protein B which were used for MS detection.
SEQ ID NO:23 is the nucleotide sequence cloned in the pET 29 vector to produce the rProtein A (HisCter).
SEQ ID NO:24 is the polypeptide sequence of rProtein A (HisCter).
SEQ ID NO:25 is the nucleotide sequence cloned in the pET 28 vector to produce the rProtein A (trx).
SEQ ID NO:26 is the polypeptide sequence of rProtein A (trx).

### FIGURES

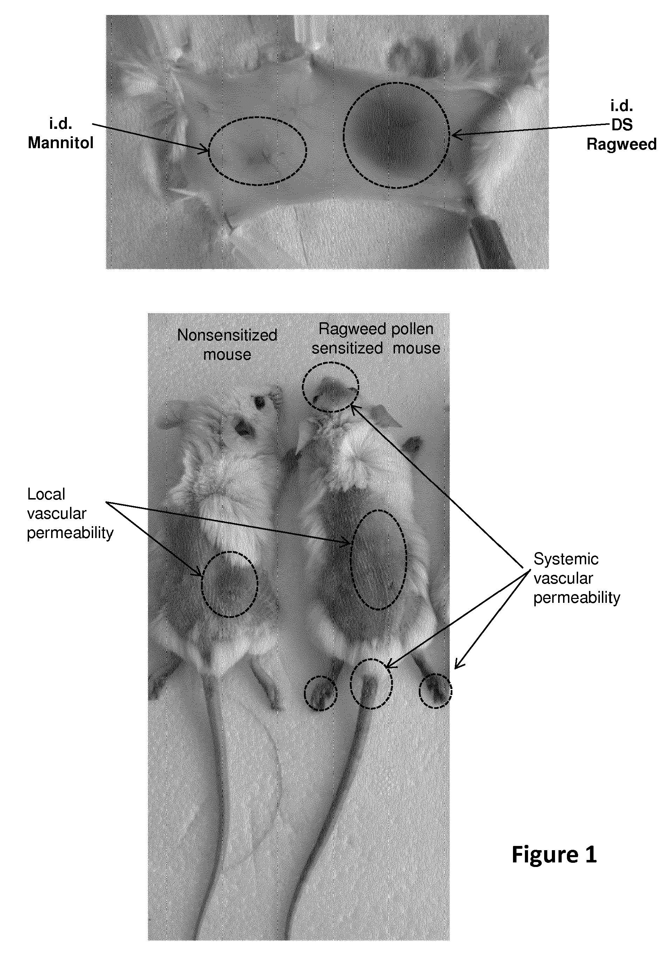

**Fig. 1****: Mouse vascular permeability assay.** Upper panel: local vascular permeability after intradermal injection showing permeability only with ragweed pollen extract. Lower panel: systemic vascular permeability in non-sensitized and ragweed pollen sensitized mouse, demonstrating that delocalized vascular permeability only in ragweed pollen sensitized mouse.

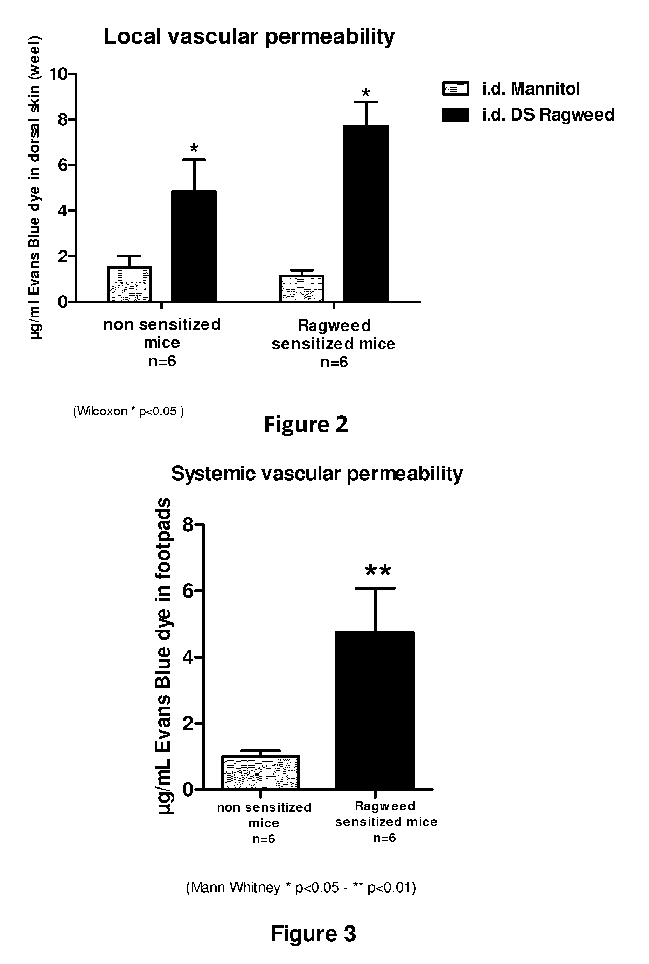

**Fig. 2****: Measurement of local vascular permeability after intradermal injection of mannitol (negative control) or ragweed pollen extract (DS ragweed), using Evans blue dye**. Ragweed pollen extract induces a local vascular permeability in both non-sensitized and ragweed sensitized mice.
**Fig. 3****: Measurement of systemic vascular permeability after intradermal injection of ragweed pollen extract (DS ragweed), using Evans blue dye.** Ragweed pollen extract induces vascular permeability in ragweed sensitized mice but not in non sensitized mice.

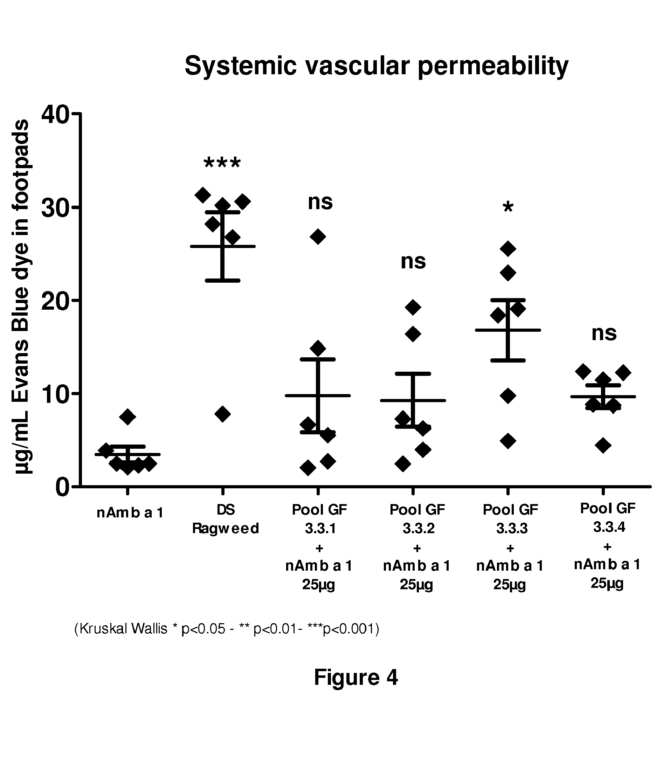

**Fig. 4****: Measurement of systemic vascular permeability after intradermal injection of chromatographic fractions (gel filtration) of ragweed pollen extract to ragweed pollen-sensitized mice.** Purified major allergen (Amb a 1, 25µg) is used as a negative control. Ragweed extract (DS ragweed) is used as a positive control. Each tested fraction contains similar amount of Amb a 1 (25µg). According to the Kruskal-Wallis statistical test and Dunn's multiple comparison test, pool GF Fraction 3.3.3 shows significantly higher systemic vascular permeability (p<0.05), as compared to other fractions as well as purified Amb a 1 major allergen. ns: not significant. Data are expressed in mean +/- SEM.

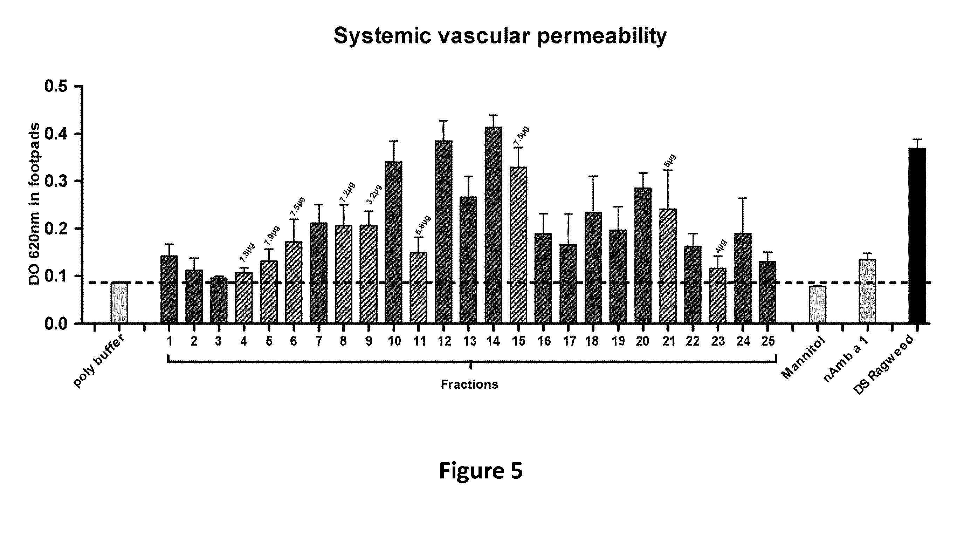

**Fig. 5****: Measurement of systemic vascular permeability after intradermal injection of chromatographic fractions (gel filtration followed by chromatofocusing) of ragweed pollen extract to ragweed pollen-sensitized mice.** Purified major allergen (Amb a 1, 25µg) alone, mannitol and poly buffer are used as negative controls. Ragweed extract (DS ragweed) is used as a positive control. Fractions 4-6, 8-9, 11, 15, 21 and 23 are tested in amounts of proteins as indicated on Fig.5, and the others at 12 µg of proteins. All tested fractions are adjusted with 25 µg of Amb a 1. Fractions 10, 12 and 14 show higher systemic vascular permeability, as compared to other fractions. Data are expressed in mean +/- SEM.
Fi**g. 6: Abundance measurements of protein A, a copper-binding-like protein, by mass spectrometry within fractions obtained by chromatographic separation of a ragweed pollen extract.** Mass spectrometry data were analyzed and quantified using a label-free strategy with LC-MS Progenesis software (Waters). Protein A quantitation was based on the intensity of four peptides (35-GWALGVDYVAWANAR-49 (SEQ ID NO:13); 50-QIVQGDELVFNYDVK-64 (SEQ ID NO:14); 65-GDFPVFYTTK-74 (SEQ ID NO:15); 80-CCPYGALMELANTGHNVVTLGGVGDYYFIPK-110 (SEQ ID NO:16). The amino acid positions are indicated by reference to SEQ ID NO:2 (precursor form of protein A). Data are expressed in mean +/- SEM.

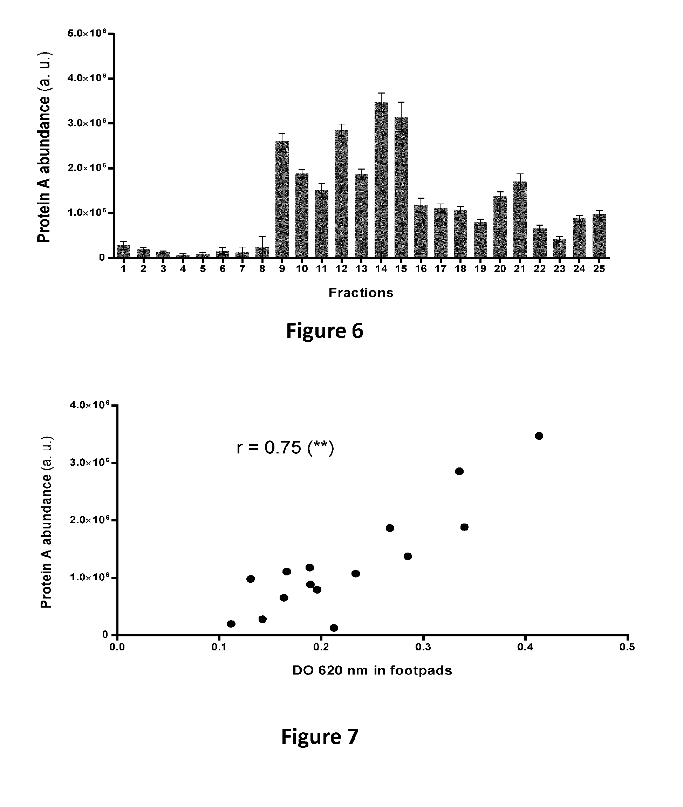

Fig. 7: **Relationship** between **(y-axis)** the abundance of protein A, a **copper-binding-like** protein, within fractions obtained by chromatographic separation of a ragweed **pollen extract,** and **(x-axis)** the intensity of vascular permeability observed in footpads of ragweed **pollen-sensitized** mice intradermally administered in their back with those fractions, as measured by the absorbance at 620 nm of solutions in which the footpad's blood was allowed to diffuse. According to the Spearman's correlation test, a significant correlation was observed between the two parameters (r = 0.75, p = 0.002).

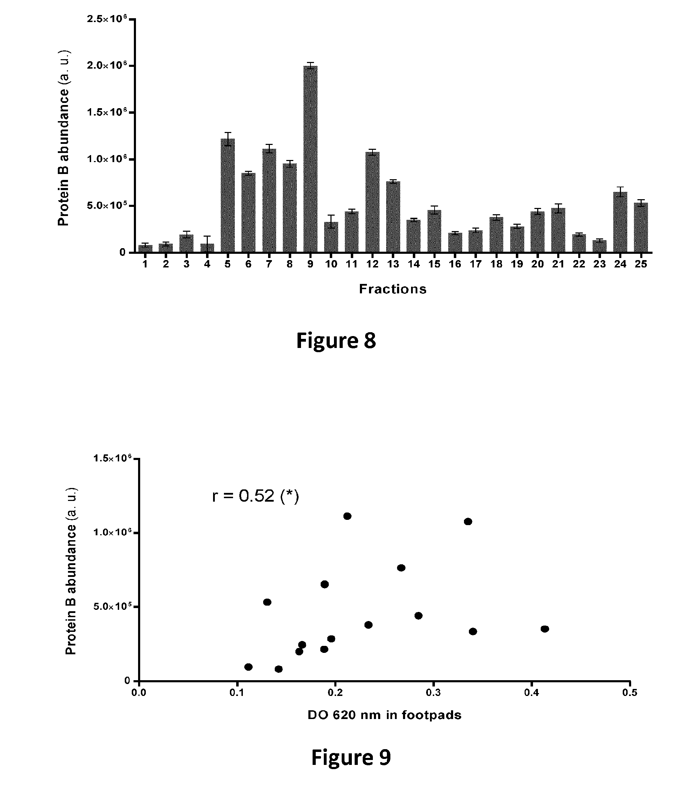

Fig. 8: Abundance measurements of protein B, a **copper-binding-like** protein, by mass spectrometry within fractions obtained by chromatographic separation of a ragweed **pollen extract.** Mass spectrometry data were analyzed and quantified using a label-free strategy with LC-MS Progenesis software (Waters). Protein B quantitation was based on the intensity of six peptides (34-GWVTGVDFEAWAK-46 (SEQ ID NO:17); 49-TFSVGDQLVFK-59 (SEQ ID NO:18); 60-YAPNYGLLLTDK-71 (SEQ ID NO:19); 60-YAPNYGLLLTDKVR-73 (SEQ ID NO:20); 81-GPILALESTGDTTVK-95 (SEQ ID NO:21); 96-IQGPGDYYFISYRPYCFEGCK-116 (SEQ ID NO:22)). The amino acid positions are indicated by reference to SEQ ID NO:5 (precursor form of protein B). Data are expressed in mean +/- SEM.
**Fig. 9****: Relationship between (*y*-axis) the abundance of protein B, a copper-binding-like protein, within fractions obtained by chromatographic separation of a ragweed pollen extract, and (*x*-axis) the intensity of vascular permeability observed in footpads of ragweed pollen-sensitized mice** intradermally administered in their back with those fractions, as measured by the absorbance at 620 nm of solutions in which the footpad's blood was allowed to diffuse. According to the Spearman's correlation test, a significant correlation was observed between the two parameters (r = 0.52, p < 0.045).

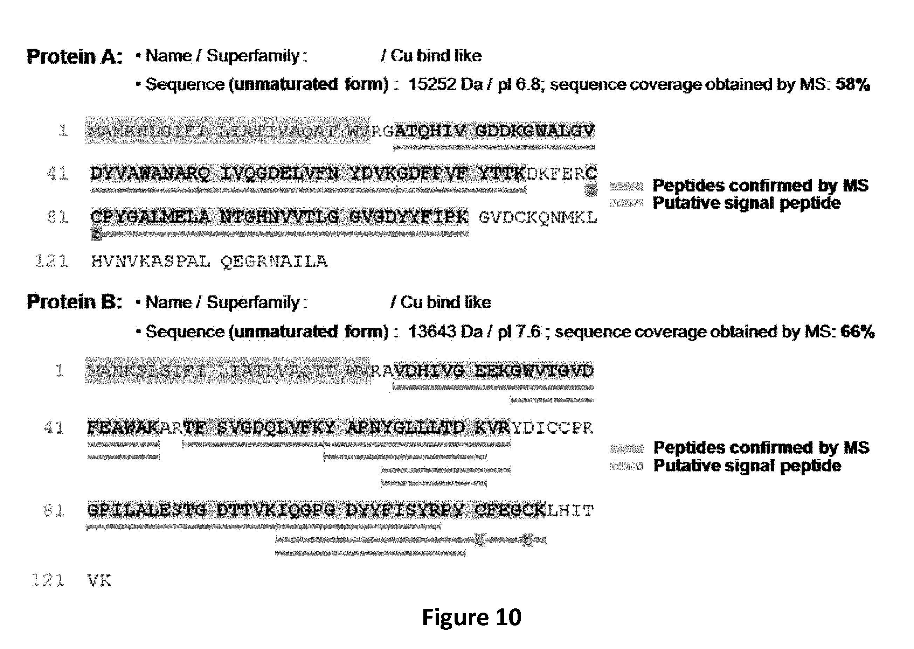

**Fig. 10****: Sequences of proteins A and B as determined by MS/MS analysis.** Amino acids in bold and highlighted in gray were confirmed by at least one conclusive MS/MS spectrum, whereas amino acids not in bold format and highlighted in grey correspond to signal peptides.

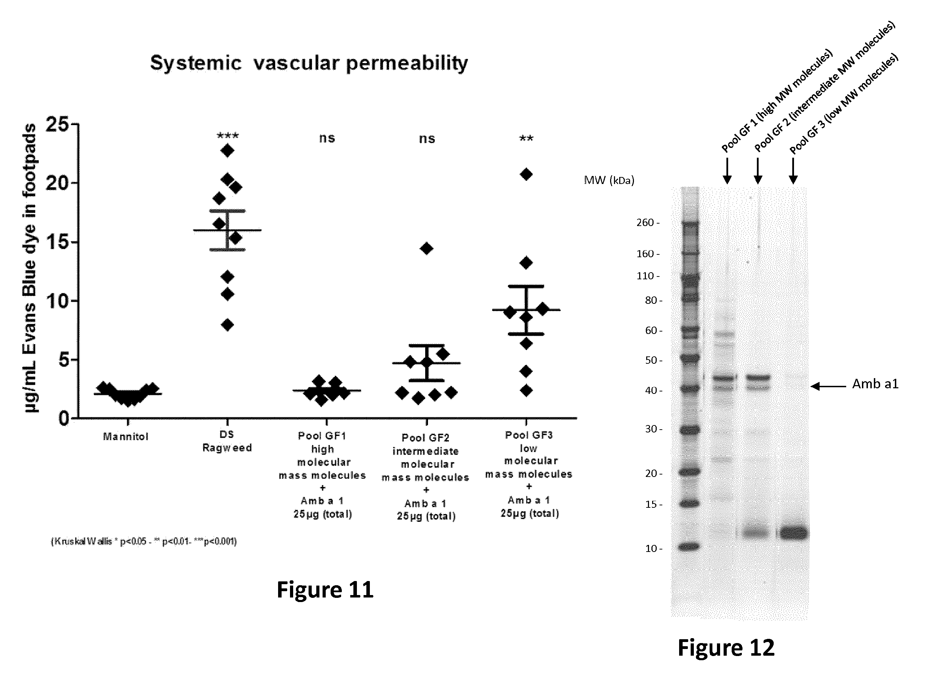

**Fig. 11****: Measurement of systemic vascular permeability after intradermal injection of chromatographic fractions (Metal Chelate affinity chromatography followed by gel filtration) of ragweed pollen extract to ragweed pollen-sensitized mice.** Mannitol is used as a negative control. Ragweed extract (DS ragweed) is used as a positive control. Each fraction is tested at 10 µg of proteins and supplemented with Amb a 1 to obtain a final dose of 25µg. According to the Kruskal-Wallis statistical test and Dunn's multiple comparison test, the vascular permeability obtained with the ragweed pollen extract and the low molecular mass molecules was significantly higher than the vascular permeability obtained with mannitol (*** p < 0.001 and ** p < 0.01, respectively). In contrast, the vascular permeability induced by the fraction of high molecular mass molecules was not significantly higher than the one obtained with mannitol (ns). Data are expressed in mean +/- SEM.
**Fig. 12****: SDS-PAGE of chromatographic fractions (Metal Chelate affinity chromatography followed by gel filtration) of ragweed pollen extract.** The gel was stained with Sypro Ruby. Metal Chelate affinity chromatography followed by gel filtration is efficacious to separate major allergens (Amb a 1 & Amb a 11, ∼45 kDa) from low molecular mass proteins (∼12 kDa proteins).

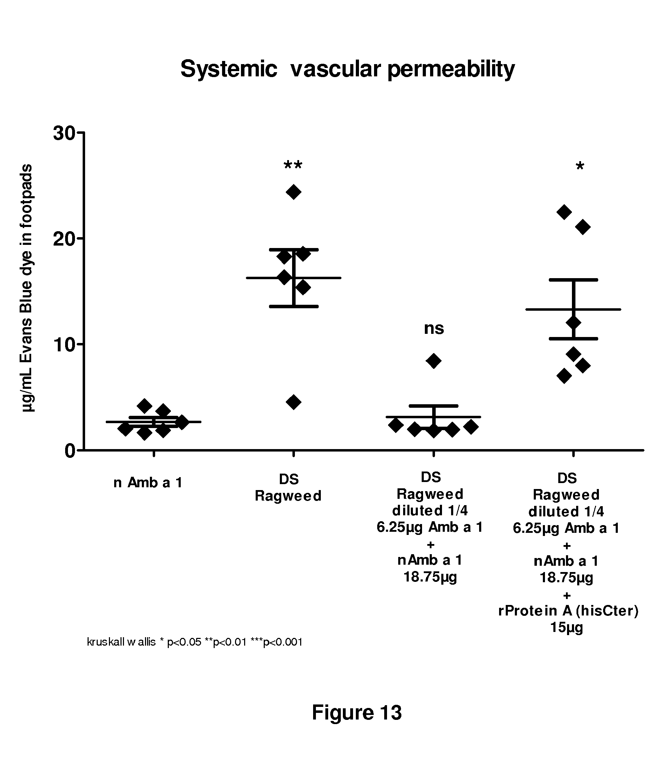

**Fig. 13****: Measurement of systemic vascular permeability after intradermal injection of the his-tagged recombinant protein A with a suboptimal dose of ragweed extract (DS ragweed diluted ¼) to ragweed pollen-sensitized mice.** Purified major allergen (Amb a 1, 25µg) alone and ¼ diluted DS ragweed adjusted with Amb a 1 to obtain a final dose of 25µg are used as negative controls. Ragweed extract (DS ragweed) is used as a positive control. According to the Kruskal-Wallis statistical test and Dunn's multiple comparison test, the addition of rProtein A (hisCter) to a suboptimal dose of ragweed extract induces significant vascular permeability (p<0.05). Data are expressed in mean +/-SEM.

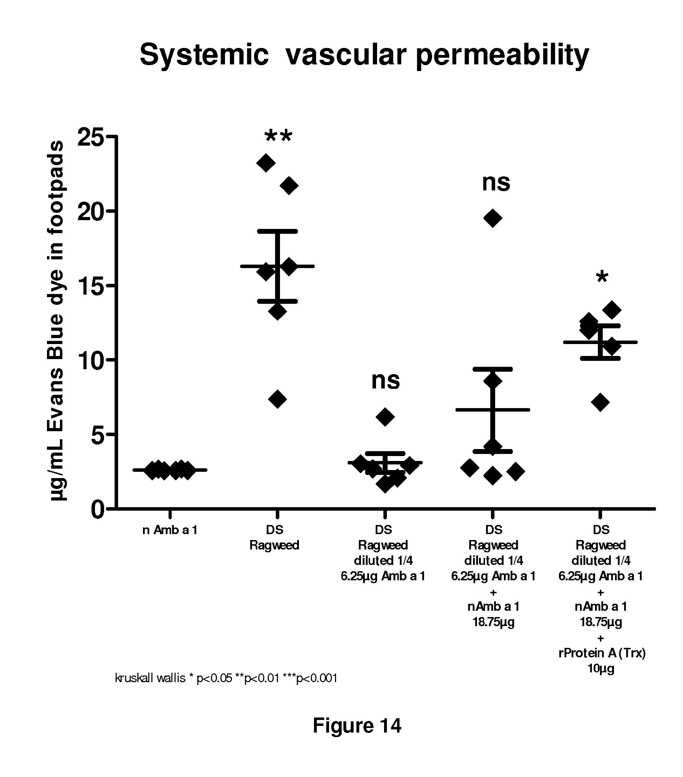

**Fig. 14****: Measurement of systemic vascular permeability after intradermal injection of the Trx recombinant protein A with a suboptimal dose of ragweed extract (DS ragweed diluted ¼) to ragweed pollen-sensitized mice.** Purified major allergen (Amb a 1, 25µg) alone, DS ragweed diluted ¼, and DS ragweed diluted supplemented with Amb a 1 to obtain a final dose of 25µg are used as negative controls. Ragweed extract (DS ragweed) is used as a positive control. According to the Kruskal-Wallis statistical test and Dunn's multiple comparison test, the addition of rProtein A (Trx) to a suboptimal dose of ragweed extract induces significant vascular permeability (p<0.05). Data are expressed in mean +/- SEM.

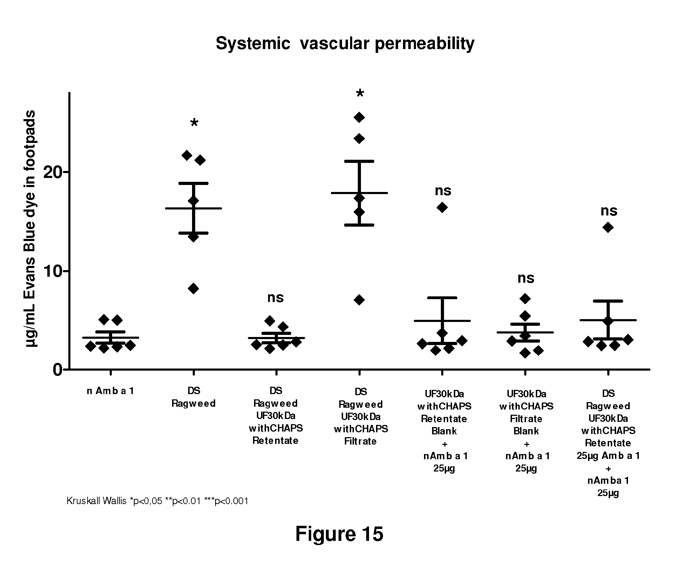

**Fig. 15****: Measurement of systemic vascular permeability after intradermal injection of ragweed pollen extract treated with CHAPS and filtrated to conserve only the protein above 30 kDa to ragweed pollen-sensitized mice.** Purified major allergen (Amb a 1, 25µg) alone is used as a negative control. Ragweed extract (DS ragweed) is used as a positive control. Each raction (retentate and filtrate after CHAPS treatment and UF 30 kDa) were tested with similar amount of Amb a 1 (25µg). Blank is a composition formulated without allergen extract. According to the Kruskal-Wallis statistical test and Dunn's multiple comparison test, the ragweed pollen extract treated with CHAPS and filtrated to conserve only the protein above 30 kDa (retentate) does not induce systemic vascular permeability, and in contrast, the filtrate induces significant permeability (p<0.05). Data are expressed in mean +/- SEM.

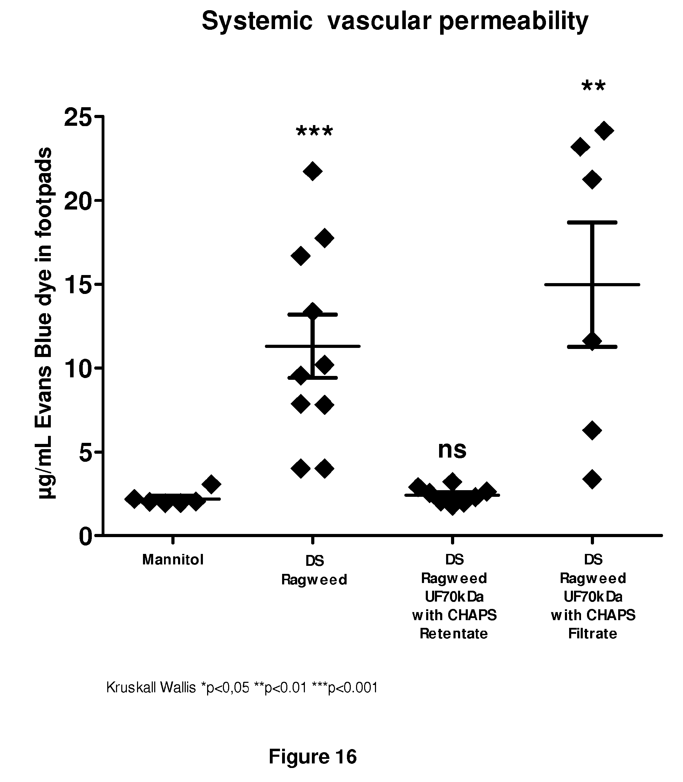

**Fig. 16****: Measurement of systemic vascular permeability after intradermal injection of ragweed pollen extract treated with CHAPS and filtrated to conserve only the protein above 70 kDa to ragweed pollen-sensitized mice.** Mannitol is used as a negative control. Ragweed extract (DS ragweed) is used as a positive control. Each tested fraction (retentate and filtrate) contains similar amount of Amb a 1 (25µg). According to the Kruskal-Wallis statistical test and Dunn's multiple comparison test, the ragweed pollen extract treated with CHAPS and filtrated to conserve only the protein above 70 kDa (retentate) does not induce systemic vascular permeability, and in contrast, the filtrate induces significant permeability (p<0.01). Data are expressed in mean +/-SEM.

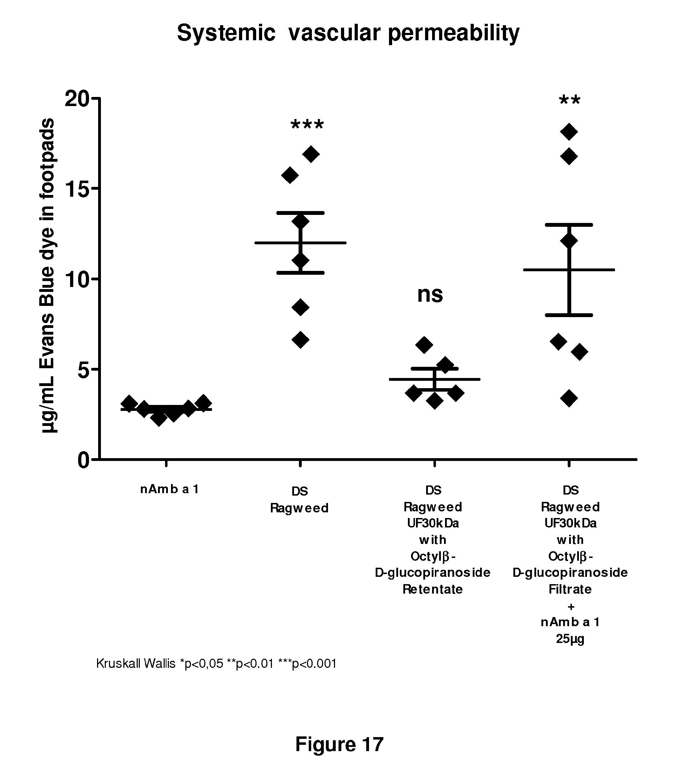

**Fig. 17****: Measurement of systemic vascular permeability after intradermal injection of ragweed pollen extract treated with Octylglucoside and filtrated to conserve only the protein above 30 kDa to ragweed pollen-sensitized mice.** Purified major allergen (Amb a 1, 25µg) alone is used as a negative control. Ragweed extract (DS ragweed) is used as a positive control. Each tested fraction (retentate and filtrate) contains similar amount of Amb a 1 (25µg). According to the Kruskal-Wallis statistical test and Dunn's multiple comparison test, the ragweed pollen extract treated with Octylglucoside and filtrated to conserve only the protein above 30 kDa (retentate) does not induce systemic vascular permeability, and in contrast, the filtrate induces significant permeability (p<0.01). In the group of "filtrate", four mice died during the experiment. Data are expressed in mean +/- SEM.

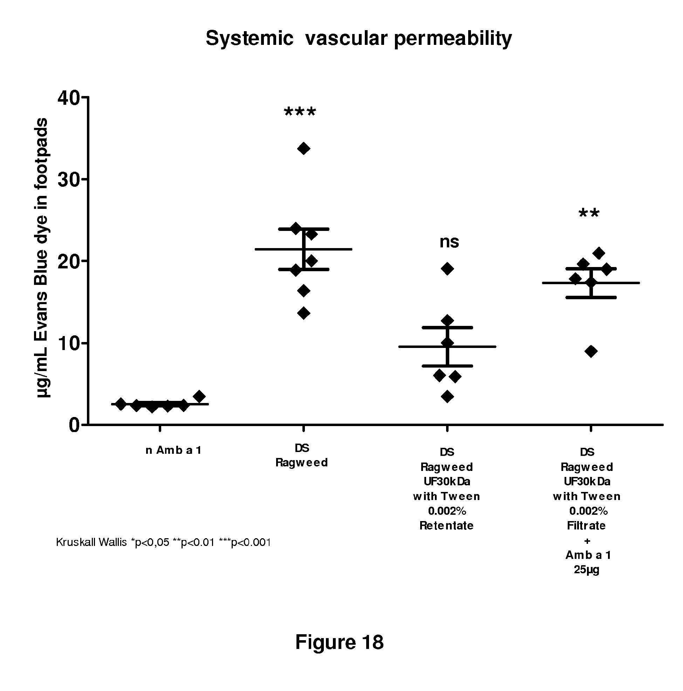

**Fig. 18****: Measurement of systemic vascular permeability after intradermal injection of ragweed pollen extract treated with Tween 80 and filtrated to conserve only the protein above 30 kDa to ragweed pollen-sensitized mice.** Purified major allergen (Amb a 1, 25µg) alone is used as a negative control. Ragweed extract (DS ragweed) is used as a positive control. Each tested fraction (retentate and filtrate) contains similar amount of Amb a 1 (25µg). According to the Kruskal-Wallis statistical test and Dunn's multiple comparison test, the systemic vascular permeability induction with the ragweed pollen extract treated with Tween 80 and filtrated to conserve only the protein above 30 kDa (retentate) doesn't induce a significant systemic vascular permeability compared to the negative control, and in contrast, the filtrate induces significant permeability (p<0.01).

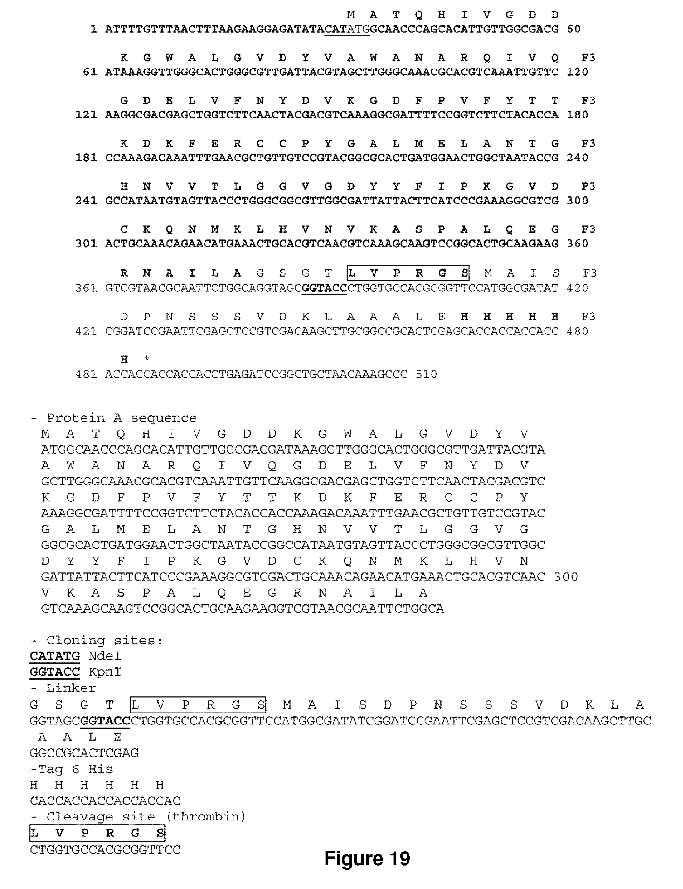

**Fig. 19****: Sequence cloned in the pET 29 vector to produce the rProtein A (HisCter).**

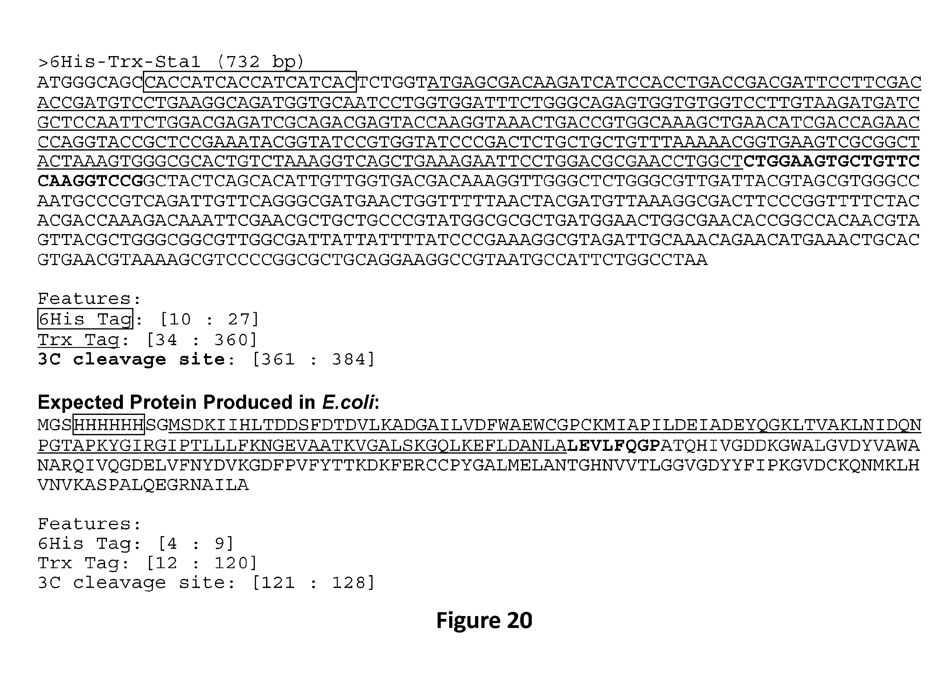

**Fig. 20****: Sequence cloned in the pET 28 vector to produce the rProtein A (trx).**

### EXAMPLES

### Example 1: occurrence of unexpected side-effects during phase I clinical trial with ragweed pollen extract

In the course of a phase I clinical trial, patients were administered with tablets containing a ragweed pollen extract or a placebo via the oral route. 10% of the patients that received the ragweed pollen extract-containing tablets experienced angioedema with unexpected features.

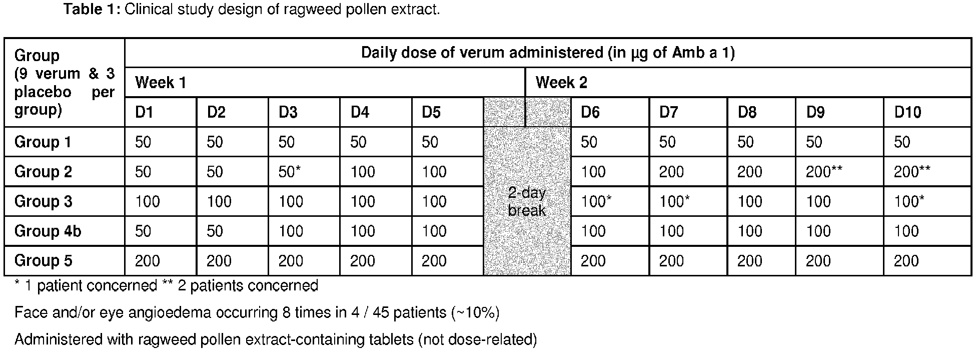

These were not typical IgE-dependent allergic reactions, since: (1) they appeared remote from the administration site (sublingual -SL); (2) they did not resolve after administration of antihistamine and corticoids; (3) their onset and (4) their duration were longer, and sometimes much longer, than expected from a typical IgE-dependent allergic reaction. It was concluded that these angioedemas were due to kinin-mediated vascular permeability.

**Table 2: The observed unexpected adverse events (AE) are due to kinin-mediated vascular permeability**

| | **Unexpected AE observed during the phase I clinical study** | **Angioedema due to kinin-mediated vascular permeability** | **Angioedema due to histamine-mediated vascular permeability** |
|---|---|---|---|
| **Response to antihistamine & corticoids** | no response | poor | excellent |
| **Onset** | mean of ∼4 h (20 min to 15 h 30) | hours | minutes |
| **Duration** | mean of ∼27 h (30 min to 5 days) | > 24 h | ≤ 24 h |

### Example 2: Mouse Vascular Permeability Protocol

In order to determine the compounds responsible for the adverse effects, a mice model of kinin-mediated, ragweed pollen-induced, increased vascular permeability was developed.

### Mice sensitization

Female BALB/c mice (6 to 8 week-old) were purchased from Charles River (L'Arbresle, France). Experimental protocols were approved by Stallergenes ethical committee for animal experimentation, and animal handling was performed according to international regulations. Mice were sensitized with 2 intra-peritoneal (i.p.) injections of Ragweed pollen extracts (using doses equivalent to 10 µg Amb a 1) on days 0 and 14 in presence of 2 mg aluminum hydroxide, then exposed from days 21 to 24 to aerosolized Ragweed pollen extracts 20 min per day, using similar doses as above.

### Vascular permeability assay

To avoid skin inflammation, dorsal hairs were carefully removed with electric clippers, two days before the vascular permeability assay (on day 25). On day 28, animals were anesthetized using sodium pentobarbital (50 mg/kg, i.p.) and xylazine (16 mg/kg) about 10-15 minutes before the injection of tested products.

Groups of six or eight mice always received an intradermal injection of mannitol (50 µL, 46 mg/mL in PBS) on the left dorsal flank. On the right dorsal flank, intradermal injection of mannitol (50 µL, 46 mg/mL in PBS) or natural purified Amb a 1 (50 µL, dose 25 µg in PBS) were used as negative controls whereas Ragweed pollen extract (50 µL, dose corresponding to 25 µg Amb a 1) was used as a positive control. Tested products (50 µL, supplemented or not with Amb a 1 to a final dose of 25µg) were administered on the right dorsal flank. Immediately after injection, Evans blue (100 µL, 1% in PBS) was administered by retro-orbital injection. After 35 min, the mice were euthanized by sodium pentobarbital overdose, skin (2 injection sites; left and right dorsal flanks) and feet were removed and placed into 0.5 mL and 1 mL of dimethylsulfoxide, respectively. The Evans blue dye was extracted from the skin and feet at +37 °C for one day (24 ± 2 hours) and measured by spectrophotometry at 620 nm using a standard range of Evans blue (0.2-250 µg Evans blue). Data were expressed in µg/mL of extravasated Evans blue, as means ± SEM. Comparisons of the concentrations of dye extravasation were evaluated by the nonparametric test (Kruskall Wallis) with GraphPad Prism. P values smaller than 0.05 were considered significant.

### Drug substance (hereinafter DS) ragweed

Pollen was incubated for 24h with a 50 mM ammonium bicarbonate solution (1 g pollen/20 mL solution) at 4 °C, under mechanical or magnetic stirring. After centrifugation at 2000 g, the supernatant was clarified using successive filtrations (polysep filter with a porosity of 1µm +/- 0.2µm). The clarified product was then clarified by tangential filtration on a membrane with a 1 kDa cut-off. This was followed by a step of diafiltration with 2.5 volumes of aqueous solution, such as water. Mannitol was then added with a 2% final ratio (w/w). A final step of filtration (0.22µm) was performed.

### Validation of the assay

The study involved 2 groups of 6 animals, with two administration sites per animal (left and right sites in the dorsal flanks).

**Table 3: Study design**

| | | Left side in the dorsal flanks | Right side in the dorsal flanks |
|---|---|---|---|
| Group 1 n=6 | Non-sensitized mice | Mannitol 2.3 mg/site in PBS (Negative control) | DS Ragweed pollen adjusted25 µg Amb a 1/site in PBS |
| Group 2 n=6 | Ragweed pollen sensitized mice | Mannitol 2.3 mg/site in PBS (Negative control) | DS Ragweed pollen adjusted 25 µg Amb a 1/site in PBS |

### Results

Intradermal administration of ragweed pollen extract induced an important increase of cutaneous vascular permeability compared to mannitol (p<0.01) in DS Ragweed pollen sensitized mice and in non-sensitized mice (Fig. 1-2). The increased local vascular permeability is higher in the DS Ragweed pollen sensitized mice group (Fig. 3).

In addition to the local effect, intradermal administration of ragweed pollen extract in ragweed pollen sensitized mice caused an increase of systemic vascular permeability, as evidenced by the blue color of the extremities (footpads, muzzle, tail). In contrast, no systemic vascular permeability was observed in non sensitized mice (Fig.1 and 3).

Further features of the unexpected adverse events observed during the phase I clinical trial of ragweed pollen extract-containing tablets were also observed in the model (Table 4).

**Table 4: Comparison of adverse events observed in clinical and in the developed mouse model**

| **Unexpected adverse events observed during the phase I clinical trial** | **Phenomenon observed in Stallergenes**'**s mouse model** |
|---|---|
| Kinin-mediated vascular permeability | Kinin-mediated vascular permeability |
| (as established from (1) the long onset of action, (2) the long duration and (3) the unresponsiveness to antihistamine and corticoid of the adverse events) | (as established from (1) the consumption of both kininogenase activity and C1 inhibitor, (2) inhibition of the phenomenon by a competitive inhibitor of kinin-mediated vascular permeability (i.e. Icatibant), (3) the similarity of the set up mouse model with a model of increased vascular permeability in C1 inhibitor-deficient mice*, and (4) the incapacity of the induction of histamine release only to trigger the observed phenomenon |
| Remote from the administration site (upper part of the face *vs* sublingual) | Remote from the administration site (extremities *vs* intra-back's skin) |

| | |
|---|---|
| * D. Han et al. The Journal of Clinical Investigation 109: 1057- 1063, April 2002 | |

Altogether, these results supported the validity of the model to further study the causes for these unexpected adverse events.

### Example 3: Assay methods

### Amb a 1 quantification assay

Amb a 1 major allergen was assessed using commercially available ELISA Kit (Indoor Biotechnologies Ltd, Warminster, United Kingdom) as per the manufacturer's instructions. Briefly, a polyclonal rabbit anti-Amb a 1 capture antibody is coated on polystyrene microtiter plates. The sample to be tested or Amb a 1 standard solution is then incubated and washed. Finally, the amount of Amb a 1 is determined using a biotinylated rabbit anti-Amb a 1 antibody.

### Example 4: Determination of compounds responsible for the angioedema

Different fractions of a ragweed pollen extract, as obtained by a two-step chromatography (gel filtration followed by ion exchange chromatography), were tested in the set-up model of kinin-mediated increased vascular permeability in ragweed pollen-sensitized mice.

In particular, a so-called pool GF 3.3.3 was isolated further to gel filtration as having most of the increased vascular permeability effect (Fig. 4).

This pool was fractionated with mono P (chromatofocusing) in 25 fractions. These fractions were first tested in the model of induced vascular permeability (Fig. 5). Fractions 10, 12 and especially the fraction 14 showed higher systemic vascular permeability, as compared to other fractions.

Then proteins from the fraction 14 were identified by nanoLC-MS/MS. The relative abundance of each protein identified was monitored by mass-spectrometry (MS) in all tested fractions and compared with vascular permeability data. Correlation between abundance of individual proteins and the level of induced vascular permeability was studied with fractions tested at the same dose (12 µg). This allowed correlating the effect on induction of vascular permeability with copper binding-like proteins, in particular with two proteins hereafter called "protein A" and "protein B" (Fig. 6-9).

### Example 5: Characterization of proteins A and B and homology with other copper binding like proteins

These proteins were identified further to the identification of tracer peptides. In the absence of a comprehensive ragweed proteome, no homology could be found with a known protein. Therefore, the transcriptome of ragweed pollen was deciphered, allowing finding hits between the tracer peptides and full length sequences.

The transcriptome analysis allowed identifying other copper-binding-like ragweed pollen-derived proteins (see Table 5).

Identity and similarity were determined against protein A using EMBOSS Needle and the following parameters:
Matrix: EBLOSUM62
gapopen 10.0
gapextend 0.5
endopen 10.0
endextend 0.5

**Table 5: Comparability of the sequence of protein A with the sequences of proteins B, C and D.**

| **Based on precursor sequences** | | | | | | |
|---|---|---|---|---|---|---|
| | **Protein B** | | **Protein C** | | **Protein D** | |
| **Length** | 140 | | 149 | | 140 | |
| **Identity** | 65/140 | 46.4% | 62/149 | 41.6% | 70/140 | 50.0% |
| **Similarity** | 83/140 | 59.3% | 78/149 | 52.3% | 83/140 | 59.3% |
| **Gaps** | 17/140 | 12.1% | 13/149 | 8.7% | 17/140 | 12.1% |

| **Based on mature sequences** | | | | | | |
|---|---|---|---|---|---|---|
| | **Protein B** | | **Protein C** | | **Protein D** | |
| **Length** | 98 | | 124 | | 115 | |
| **Identity** | 44/115 | 38.3% | 40/124 | 32.3% | 47/115 | 40.9% |
| **Similarity** | 60/115 | 52.2% | 55/124 | 44.4% | 59/115 | 51.3% |
| **Gaps** | 17/115 | 14.8% | 13/124 | 10.5% | 17/115 | 14.8% |

### Example 6: Induction of vascular permeability by ragweed extract after molecular size-based separation

The effect of these proteins was further confirmed after molecular size-based separation with gel filtration. Indeed, the vascular permeability obtained with the ragweed pollen extract and the lowest molecular mass molecules thereof (<15kDa) was significantly greater than the vascular permeability obtained with mannitol, whereas vascular permeability induced by the fraction of low molecular mass molecules (15 to 45kDa) and high molecular mass molecules (45 to 75 kDa) were not significantly greater than the one obtained with mannitol (Fig. 11 and 12).

In contrast, no correlation was observed for any other protein detected in those fractions, such as the relevant ragweed pollen allergen Amb a 1.

### Example 7: production of recombinant Protein A

### Production of His-tagged recombinant protein A (rProtein A (HisCter))

A sequence encoding a HisCTer-ProteinA (Fig. 19) was cloned in pET 29 vector.

The resulting plasmids were used to transform E. coli BL21 (DE3) strains. Overnight starter cultures grown at 37 °C in LB medium were diluted in fresh medium and further grown until reaching an OD 600nm of 0.4-0.8. Recombinant protein expression was then induced for 4 h with 1 m M IPTG. Cell pellets were collected by centrifugation and stored at -20 °C until use.

Cell pellets were subjected to 5 freeze/thaw cycles, resuspended in 50 m M Tris pH8, 50 m M NaCl, 1 m M EDTA, incubated on a tube rotator for 30 min at 4 °C and then sonicated. Inclusion bodies were recovered by centrifugation.

Inclusion bodies were washed and subsequently solubilised overnight with 8 M urea at 4 °C. Refolding was performed by overnight dialysis against PBS buffer. Refolded proteins were then concentrated using centrifugal devices with 3kDa MWCO.

### Production and processing of TRX-proteinA fusion protein (rProtein A (Trx))

A sequence encoding a TRX-protein A fusion with 3C-protease cleavage site (Fig. 20) was cloned in pET 28 vector

The resulting plasmids were used to transform E. coli. Cells were grown at 37 °C until OD600nm reaches 0.5. Fusion protein expression was done at 12 °C for 16h with 1 mM IPTG. Cell lysate was clarified by centrifugation and soluble fraction was collected. TRX-proteinA was immobilized in native conditions on a nickel affinity chromatography column. TRX fusion partner cleavage was then done with 3C-protease for 16h in cleavage buffer (50mM Tris-HCl pH 7, 150mM NaCl, 1mMEDTA, 1 mM DTT). Recombinant protein A was then eluted in cleavage buffer.

### Example 8: Induction of vascular permeability by rProtein A (HisCter)

Experiments were conducted in the model of induced vascular permeability with rProtein A (HisCter).

The first study involved 4 groups of 6 animals, with two administration sites per animal (left and right site in the dorsal flanks). The study design was as described in Table 6.

**Table 6: Study design**

| | | Left side in the dorsal flanks | Right side in the dorsal flanks |
|---|---|---|---|
| Group 1 | Ragweed pollen sensitized mice | Mannitol 2.3 mg/site in PBS (Negative control) | nAmb a 1 (25 µg Amb a 1/site in PBS) (Negative control) |
| n=6 | | | |
| Group 2 | Ragweed pollen sensitized mice | Mannitol 2.3 mg/site in PBS (Negative control) | DS Ragweed pollen (25 µg Amb a 1/site in PBS) (Positive control) |
| n=6 | | | |
| Group 3 | Ragweed pollen sensitized mice | Mannitol 2.3 mg/site in PBS (Negative control) | DS Ragweed pollen diluted 1/4 (6.25 µg Amb a 1/site in PBS) with Amb a 1 (18.75 µg/site in PBS) to adjust Amb a 1 to 25 µg Amb a 1/site (Negative control) |
| n=6 | | | |
| Group 4 | Ragweed pollen sensitized mice | Mannitol 2.3 mg/site in PBS (Negative control) | rProtein A (HisCter) (15µg/site in PBS) with DS Ragweed pollen diluted 1/4 (6.25 µg Amb a 1/site in PBS) with Amb a 1 (18.75 µg/site in PBS)) to adjust Amb a 1 to 25 µg Amb a 1/site |
| n=6 | | | |

| | | | |
|---|---|---|---|
| rProtein A (HisCter), with a suboptimal dose of ragweed extract, increased significantly vascular permeability (Fig. 13, p<0.05). This confirmed the involvment of Protein A in the induction of vascular permeability. | | | |

### Example 9: Induction of vascular permeability by rProtein A (Trx)

Experiments were conducted in the model of induced vascular permeability with rProtein A (Trx).

The study involved 5 groups of 6 animals, with two administration sites per animal (left and right site in the dorsal flanks). The study design was as described in Table 7.

**Table 7: Study design**

| | | Left side in the dorsal flanks | Right side in the dorsal flanks |
|---|---|---|---|
| Group 1 | Ragweed pollen sensitized mice | Mannitol 2.3 mg/site in PBS (Negative control) | nAmb a 1 25 µg Amb a 1/site in PBS (Negative control) |
| n=6 | | | |
| Group 2 | Ragweed pollen sensitized mice | Mannitol 2.3 mg/site in PBS (Negative control) | DS Ragweed pollen 25 µg Amb a 1/site in PBS (Positive control) |
| n=6 | | | |
| Group 3 | Ragweed pollen sensitized mice | Mannitol 2.3 mg/site in PBS (Negative control) | DS Ragweed pollen diluted (1/4) 6.25 µg Amb a 1/site in PBS (Negative control) |
| n=6 | | | |
| Group 4 | Ragweed pollen sensitized mice | Mannitol 2.3 mg/site in PBS (Negative control) | DS Ragweed pollen diluted (1/4) (6.25 µg Amb a 1/site in PBS) with Amb a 1 (18.75 µg/site in PBS) to adjust Amb a 1 to 25 µg Amb a 1/site (Negative control) |
| n=6 | | | |
| Group 5 | Ragweed pollen sensitized mice | Mannitol 2.3 mg/site in PBS (Negative control) | rProtein A (Trx) (10µg/site in PBS) with DS Ragweed pollen diluted (1/4) (6.25 µg Amb a 1/site in PBS) with Amb a 1 (18.75 µg/site in PBS)) to adjust Amb a 1 to 25 µg Amb a 1/site |
| n=5* | | | |

| | | | |
|---|---|---|---|
| *One mouse died during the experiment | | | |

The results shown on Fig. 14 demonstrate that the addition of rProtein A (Trx), with a suboptimal dose of ragweed pollen extract, increased significantly vascular permeability (p<0.05). This confirmed the role of Protein A obtained with rProtein A (HisCter) (Fig 15) in the induction of vascular permeability.

### Example 10: Methods of depletion of copper-binding-like proteins - CHAPS - UF 30kDa

Pollen was incubated for 24h with a 50 mM ammonium bicarbonate solution (1 g pollen/20 mL solution) at 4 °C, under magnetic stirring. After centrifugation at 2000 g, the supernatant was clarified using successive filtrations.

3-[(3-cholamidopropyl)dimethylammonio]-1-propanesulfonate (CHAPS, 1.63mM final concentration) was incubated for 60 minutes with the clarified supernatant under magnetic stirring at room temperature. This preparation was concentrated by ultrafiltration (UF/DF) using a 30 kDa membrane (PM-30, PALL) with a concentration volumic factor of 10 fold. Then, the retentate was dialysed against phosphate buffered saline (PBS) with a 10 kDa membrane (PM-10, Millipore). Finally, the filtrate was concentrated using an Amicon PM-5 membrane (5 kDa), with a concentration volumic factor of 10 fold and dialysed in PBS with a PM-5 membrane.

Filtrate and retentate were tested in the model of induced vascular permeability.The study involved 7 groups of 6-5 animals, with two administration sites per animal (left and right site in the dorsal flanks). The study design was as described in Table 8.

**Table 8: Study design**

| | | Left side in the dorsal flanks | Right side in the dorsal flanks |
|---|---|---|---|
| Group 1 | Ragweed pollen sensitized mice | Mannitol 2.3 mg/site in PBS (Negative control) | nAmb a 1 (25 µg Amb a 1/site in PBS) (Negative control) |
| n=6 | | | |
| Group 2 | Ragweed pollen sensitized mice | Mannitol 2.3 mg/site in PBS (Negative control) | DS Ragweed pollen (25 µg Amb a 1/site in PBS) (Positive control) |
| n=5* | | | |
| Group 3 | Ragweed pollen sensitized mice | Mannitol 2.3 mg/site in PBS (Negative control) | DS Ragweed UF30kDa with CHAPS Retentate (25 µg Amb a 1/site in PBS) |
| n=6 | | | |
| Group 4 | Ragweed pollen sensitized mice | Mannitol 2.3 mg/site in PBS (Negative control) | DS Ragweed UF30kDa with CHAPS Filtrate (25 µg Amb a 1/site in PBS) |
| n=5* | | | |
| Group 5 | Ragweed pollen sensitized mice | Mannitol 2.3 mg/site in PBS (Negative control) | UF30kDa with CHAPS Retentate blank with Amb a 1 (25 µg Amb a 1/site in PBS) |
| n=6 | | | |
| Group 6 | Ragweed pollen sensitized mice | Mannitol 2.3 mg/site in PBS (Negative control) | UF30kDa with CHAPS filtrate blank with Amb a 1 (25 µg Amb a 1/site in PBS) |
| n=6 | | | |
| Group 7 | Ragweed pollen sensitized mice | Mannitol 2.3 mg/site in PBS (Negative control) | DS Ragweed UF30kDa with CHAPS Retentate (25 µg Amb a 1/site in PBS) with additional Amb a 1 25µg |
| n=6 | | | |

| | | | |
|---|---|---|---|
| *One mouse died during the experiment | | | |

UF/DF 30 kDa with 0.1% CHAPS (3-[(3-cholamidopropyl)dimethylammonio]-1-propanesulfonate) allowed to deplete the composition from the compounds inducing vascular permeability and showed a decrease of vascular permeability (Fig.15). The use of CHAPS, a surfactant, allowed disruption of protein complexes.

As regards yield, 63% of Amb a 1 was retained in the retentate with a total allergenic activity of 0.42 IR/µg (Amb a 1).

### Example 11: Methods of depletion of copper-binding-like proteins - CHAPS - UF 70kDa

Pollen was incubated for 24h with a 50 mM ammonium bicarbonate solution (1 g pollen/20 mL solution) at 4 °C, under magnetic stirring. After centrifugation at 2000 g, the supernatant was clarified using successive filtrations.

3-[(3-cholamidopropyl)dimethylammonio]-1-propanesulfonate (CHAPS, 1.63mM final concentration) was incubated for 60 minutes with the clarified supernatant under magnetic stirring at room temperature. This preparation was concentrated by ultrafiltration (UF/DF) using a 70 kDa membrane (PM-70, PALL) with a concentration volumic factor of 10 fold. Then, the retentate was dialysed against phosphate buffered saline (PBS) with a 10 kDa membrane (PM-10, Millipore). Finally, the filtrate was concentrated using an Amicon PM-5 membrane (5 kDa), with a concentration volumic factor of 10 fold and dialysed in PBS with a PM-5 membrane.

Filtrate and retentate were tested in the model of induced vascular permeability.The study involved 4 groups of 6-10 animals, with two administration sites per animal (left and right site in the dorsal flanks). The study design was as described in Table 9.

**Table 9: Study design**

| | | Left side in the dorsal flanks | Right side in the dorsal flanks |
|---|---|---|---|
| Group 1 | Ragweed pollen sensitized mice | Mannitol 2.3 mg/site in PBS (Negative control) | Mannitol 2.3 mg/site in PBS (Negative control) |
| n=6 | | | |
| Group 2 | Ragweed pollen sensitized mice | Mannitol 2.3 mg/site in PBS (Negative control) | DS Ragweed pollen (25 µg Amb a 1/site in PBS) (Positive control) |
| n=10 | | | |
| Group 3 | Ragweed pollen sensitized mice | Mannitol 2.3 mg/site in PBS (Negative control) | DS Ragweed UF70kDa with CHAPS Retentate (25 µg Amb a 1/site in PBS) |
| n=8 | | | |
| Group 4 | Ragweed pollen sensitized mice | Mannitol 2.3 mg/site in PBS (Negative control) | DS Ragweed UF70kDa with CHAPS Filtrate (25 µg Amb a 1/site in PBS) |
| n=6 | | | |

UF/DF 70 kDa with 0.1% CHAPS (3-[(3-cholamidopropyl)dimethylammonio]-1-propanesulfonate) allowed to deplete the composition from the compounds inducing vascular permeability and showed a decrease of vascular permeability (Fig.16). The use of CHAPS, a surfactant, allowed disruption of protein complexes.

As regards yield, 59% of Amb a 1 was retained in the retentate with a total allergenic activity of 0.12 IR/µg (Amb a 1).

### Example 13: Methods of depletion of copper-binding-like proteins-octyl β-D-glucopyranoside - UF 30kDa

Pollen was incubated for 24h with a 50 mM ammonium bicarbonate solution (1 g pollen/20 mL solution) at 4 °C, under magnetic stirring. After centrifugation at 2000 g, the supernatant was clarified using successive filtrations.

Octyl β-D-glucopyranoside (0.1% w/w final concentration) was incubated for 60 minutes with the clarified supernatant under magnetic stirring at room temperature. This preparation was concentrated by ultrafiltration (UF/DF) using a 30 kDa membrane (PM-30 membrane, PALL) with a concentration volumic factor of 11 fold. Then, the retentate was dialysed against phosphate buffered saline (PBS) with a 10 kDa membrane (PM-10, Millipore). Finally, the filtrate was concentrated using an Amicon PM-5 membrane (5 kDa), with a concentration volumic factor of 10 fold and dialysed in PBS with a PM-5 membrane.

Filtrate and retentate were tested in the model of induced vascular permeability. The study involved 4 groups of 6 animals, with two administration sites per animal (left and right site in the dorsal flanks). The study design was as described in Table 10.

**Table 10: Study design**

| | | Left side in the dorsal flanks | Right side in the dorsal flanks |
|---|---|---|---|
| Group 1 | Ragweed pollen sensitized mice | Mannitol 2.3 mg/site in PBS (Negative control) | nAmb a 1 (25 µg Amb a 1/site in PBS) (Negative control) |
| n=6 | | | |
| Group 2 | Ragweed pollen sensitized mice | Mannitol 2.3 mg/site in PBS (Negative control) | DS Ragweed pollen (25 µg Amb a 1/site in PBS) (Positive control) |
| n=10 | | | |
| Group 3 | Ragweed pollen sensitized mice | Mannitol 2.3 mg/site in PBS (Negative control) | DS Ragweed UF30KDa with Octyl glucoside Retentate (25 µg Amb a 1/site in PBS) |
| n=8 | | | |
| Group 4 | Ragweed pollen sensitized mice | Mannitol 2.3 mg/site in PBS (Negative control) | DS Ragweed UF30KDa with Octyl glucoside Filtrate) with Amb a 1 (to adjust Amb a 1 to 25 µg Amb a 1/in PBS |
| n=6* | | | |

| | | | |
|---|---|---|---|
| *4 mice died during the experiment (at the end) | | | |

UF/DF 30 kDa with 0.1% Octyl β-D-glucopyranoside allowed to deplete the composition from the compounds inducing vascular permeability and showed a decrease of vascular permeability (Fig. 17). The use of Octyl β-D-glucopyranoside, a surfactant, allowed disruption of protein complexes.

As regards yield, 47% of Amb a 1 was retained in the retentate.

### Example 14: Methods of depletion of copper-binding-like proteins - Polyoxyethylenesorbitan monooleate or Tween® 80 - UF 30kDa

Pollen was incubated for 24h with a 50 mM ammonium bicarbonate solution (1 g pollen/20 mL solution) at 4 °C, under magnetic stirring. After centrifugation at 2000 g, the supernatant was clarified using successive filtrations.

Polyoxyethylenesorbitan monooleate or Tween@ 80 (0,012mM final concentration or 0.002% w/w final concentration) was incubated for 60 minutes with the clarified supernatant under magnetic stirring at room temperature. This preparation was concentrated by ultrafiltration (UF/DF) using a 30 kDa membrane (PM-30, PALL) with a concentration volumic factor of 4 fold. Then, the retentate was diafiltrated against phosphate buffered saline (PBS) with a 30 kDa membrane (PM-30, PALL). Finally, the filtrate was concentrated using a 5 kDa membrane (PM-5, Amicon), with a concentration volumic factor of 10 fold and dialysed in PBS with a 5kDa membrane (PM-5, Millipore).

Filtrate and retentate were tested in the model of induced vascular permeability. The study involved 4 groups of 6 animals, with two administration sites per animal (left and right site in the dorsal flanks). The study design was as described in Table 11.

**Table 11: Study design**

| | | Left side in the dorsal flanks | Right side in the dorsal flanks |
|---|---|---|---|
| Group 1 | Ragweed pollen sensitized mice | Mannitol 2.3 mg/site in PBS (Negative control) | nAmb a 1 (25 µg Amb a 1/site in PBS) (Negative control) |
| n=6 | | | |
| Group 2 | Ragweed pollen sensitized mice | Mannitol 2.3 mg/site in PBS (Negative control) | DS Ragweed pollen (25 µg Amb a 1/site in PBS) (Positive control) |
| n=7 | | | |
| Group 3 | Ragweed pollen sensitized mice | Mannitol 2.3 mg/site in PBS (Negative control) | DS Ragweed UF30KDa with Tween Retentate (25 µg Amb a 1/site in PBS) |
| n=6 | | | |
| Group 4 | Ragweed pollen sensitized mice | Mannitol 2.3 mg/site in PBS (Negative control) | DS Ragweed UF30KDa with Tween Filtrate) with Amb a 1 (to adjust Amb a 1 to 25 µg Amb a 1/site in PBS |
| n=6 | | | |

UF/DF 30 kDa with 0.002% Tween allowed to deplete the composition from the compounds inducing vascular permeability and showed a decrease of vascular permeability (Fig. 18). The use of Tween, a surfactant, allowed disruption of protein complexes.
As regards yield, 63% of Amb a 1 was retained in the retentate with a total allergenic activity of 1.16 IR/µg (Amb a 1).

## Claims

1. A method of preparing a purified allergen extract, which method comprises the step consisting of reducing the amount of copper binding-like proteins in the allergen extract.

2. The method according to claim 1, wherein the step consisting of reducing the amount of copper binding-like proteins in the extract is performed by filtration, size exclusion, ion-exchange, hydrophobic interaction, affinity or mixed mode chromatography, or phase separation.

3. The method according to claim 1 or 2, wherein the step consisting of reducing the amount of copper binding-like proteins in the allergen extract is performed by ultrafiltration on a 10-70 kDa membrane.

4. The method according to claim 3, wherein a surfactant is added to the allergen extract before ultrafiltration.

5. The method according to claim 4, wherein the surfactant is selected from the group consisting of CHAPS (3-[(3-cholamidopropyl)dimethylammonio]-1-propanesulfonate), Tween@ 80 (Polyoxyethylenesorbitan monooleate), octylβglucosides, or a combination thereof.

6. The method according to any one of claims 1 or 5, wherein the amount of at least one copper binding-like protein which has at least 30% sequence identity with the protein consisting of sequence SEQ ID NO:3 is reduced.

7. The method according to claim 6, wherein said at least one copper binding-like protein is (i) a protein consisting of SEQ ID NO:2, or a protein comprising or consisting of SEQ ID NO:3, (ii) and/or a protein consisting of SEQ ID NO:5, or a protein comprising or consisting of SEQ ID NO:6, (iii) and/or a protein consisting of SEQ ID NO:8, or a protein comprising or consisting of SEQ ID NO:9, (iv) and/or a a protein consisting of SEQ ID NO:11, or a protein comprising or consisting of SEQ ID NO:12.

8. The method according to any one of claims 1 or 7, wherein the allergen content of the purified allergen extract is reduced by less than 60% compared to the initial content of the allergen extract.

9. The method according to any one of claims 1 or 8, wherein said allergen extract comprises ragweed allergen extract.

10. The method according to any one of claims 1 or 9, which further comprises the step of formulating said purified allergen extract into a pharmaceutical composition.

11. A purified allergen extract which is obtainable by the method according to any one of claims 1 or 9.

12. A pharmaceutical composition which is obtainable by the method according to claim 10.

13. A solid pharmaceutical composition containing a purified ragweed pollen extract in which copper binding-like proteins are in amounts such that they do not induce vascular permeability.

14. The composition according to claim 13, wherein the induction of vascular permeability is determined according to a model of kinin-mediated vascular permeability model and is not higher than that induced by a control.

15. A composition according to any one of claims 10 or 13, for use for allergy treatment and/or prevention, wherein said allergy treatment and/or prevention is associated with reduced risk of vascular permeability.

16. An isolated protein comprising a sequence consisting of SEQ ID NO:3, SEQ ID NO:6, SEQ ID NO:9 or SEQ ID NO:12, or of a sequence at least 40% identical to any of SEQ ID NO:3, SEQ ID NO:6, SEQ ID NO:9 or SEQ ID NO:12.

17. The isolated protein according to claim 16 which comprises a sequence at least 90% identical to any of SEQ ID NO:3, SEQ ID NO:6, SEQ ID NO:9 or SEQ ID NO:12.

18. The isolated protein according to claim 16 or 17, wherein said protein is a copper binding like protein and/or induces kinin-mediated vascular permeability when administered to a Mammal.

19. A nucleic acid sequence comprising a polynucleotide sequence encoding a protein according to any of claims 16 to 18.

20. An isolated antibody which specifically binds to a protein according to any of claims 16 to 18.

21. An aptamer which specifically binds to a protein according to any of claims 16 to 18.

22. A method of detecting or quantifying copper binding-like proteins in a ragweed pollen extract, which comprises the steps consisting of:
a) contacting a ragweed pollen extract with antibodies according to claim 20 or an aptamer according to claim 21;
b) detecting immune complexes formed between the antibodies or aptamers and ligands present in the short ragweed pollen extract
c) deducing from the complexes detected the presence, absence or quantity of ragweed copper binding-like proteins in the short ragweed pollen extract.

23. A method of depleting in a ragweed pollen extract from at least one ragweed copper binding-like protein which comprises the steps consisting of:
a) contacting a ragweed pollen extract with antibodies according to claim 20 or an aptamer according to claim 21; and
b) removing immune complexes formed between the antibodies or aptamers and ligands present in the ragweed pollen extract.


SEQUENCE LISTING

<110>  Stallergenes S.A.

<120>  COMPOSITION OF ALLERGEN EXTRACTS HAVING REDUCED TOXICITY AND
       METHOD OF PRODUCTION THEREOF

<130>  BET 14P1059

<160>  26

<170>  PatentIn version 3.5

<210>  1
<211>  417
<212>  DNA
<213>  Ambrosia artemisiifolia


<220>
<221>  sig_peptide
<222>  (1)..(72)

<220>
<221>  CDS
<222>  (1)..(417)

<400>  1
atg gct aac aaa aat ttg ggc atc ttc atc ctc ata gca aca atc gta      48
Met Ala Asn Lys Asn Leu Gly Ile Phe Ile Leu Ile Ala Thr Ile Val
1               5                   10                  15

gct cag gct aca tgg gtt cga ggg gca acg cag cat ata gtt gga gac      96
Ala Gln Ala Thr Trp Val Arg Gly Ala Thr Gln His Ile Val Gly Asp
            20                  25                  30

gat aaa ggc tgg gcc ctg ggt gtt gat tac gta gct tgg gcc aat gcg      144
Asp Lys Gly Trp Ala Leu Gly Val Asp Tyr Val Ala Trp Ala Asn Ala
        35                  40                  45

agg caa atc gtc cag gga gat gaa ctt gtg ttc aat tac gat gtg aaa      192
Arg Gln Ile Val Gln Gly Asp Glu Leu Val Phe Asn Tyr Asp Val Lys
    50                  55                  60

caa gac ttt ccc gta ttt tac acg act aaa gat aaa ttt gaa cga tgt      240
Gln Asp Phe Pro Val Phe Tyr Thr Thr Lys Asp Lys Phe Glu Arg Cys
65                  70                  75                  80

tgt cct tat gga gca tta atg gaa ttg gct aat act gga cat aat gtg      288
Cys Pro Tyr Gly Ala Leu Met Glu Leu Ala Asn Thr Gly His Asn Val
                85                  90                  95

gtc acc tta gga ggc gtg ggc gac tat tat ttt att cct aaa gga gtt      336
Val Thr Leu Gly Gly Val Gly Asp Tyr Tyr Phe Ile Pro Lys Gly Val
            100                 105                 110

gat tgt aaa caa aat atg aag ctt cat gtc aat gtc aag gca agt cct      384
Asp Cys Lys Gln Asn Met Lys Leu His Val Asn Val Lys Ala Ser Pro
        115                 120                 125

gca tta cag gag gga cga aac gcc ata ctg gca                          417
Ala Leu Gln Glu Gly Arg Asn Ala Ile Leu Ala
    130                 135

<210>  2
<211>  139
<212>  PRT
<213>  Ambrosia artemisiifolia

<400>  2

Met Ala Asn Lys Asn Leu Gly Ile Phe Ile Leu Ile Ala Thr Ile Val
1               5                   10                  15

Ala Gln Ala Thr Trp Val Arg Gly Ala Thr Gln His Ile Val Gly Asp
            20                  25                  30

Asp Lys Gly Trp Ala Leu Gly Val Asp Tyr Val Ala Trp Ala Asn Ala
        35                  40                  45

Arg Gln Ile Val Gln Gly Asp Glu Leu Val Phe Asn Tyr Asp Val Lys
    50                  55                  60

Gln Asp Phe Pro Val Phe Tyr Thr Thr Lys Asp Lys Phe Glu Arg Cys
65                  70                  75                  80

Cys Pro Tyr Gly Ala Leu Met Glu Leu Ala Asn Thr Gly His Asn Val
                85                  90                  95

Val Thr Leu Gly Gly Val Gly Asp Tyr Tyr Phe Ile Pro Lys Gly Val
            100                 105                 110

Asp Cys Lys Gln Asn Met Lys Leu His Val Asn Val Lys Ala Ser Pro
        115                 120                 125

Ala Leu Gln Glu Gly Arg Asn Ala Ile Leu Ala
    130                 135


<210>  3
<211>  115
<212>  PRT
<213>  Ambrosia artemisiifolia

<400>  3

Ala Thr Gln His Ile Val Gly Asp Asp Lys Gly Trp Ala Leu Gly Val
1               5                   10                  15

Asp Tyr Val Ala Trp Ala Asn Ala Arg Gln Ile Val Gln Gly Asp Glu
            20                  25                  30

Leu Val Phe Asn Tyr Asp Val Lys Gln Asp Phe Pro Val Phe Tyr Thr
        35                  40                  45

Thr Lys Asp Lys Phe Glu Arg Cys Cys Pro Tyr Gly Ala Leu Met Glu
    50                  55                  60

Leu Ala Asn Thr Gly His Asn Val Val Thr Leu Gly Gly Val Gly Asp
65                  70                  75                  80

Tyr Tyr Phe Ile Pro Lys Gly Val Asp Cys Lys Gln Asn Met Lys Leu
                85                  90                  95

His Val Asn Val Lys Ala Ser Pro Ala Leu Gln Glu Gly Arg Asn Ala
            100                 105                 110

Ile Leu Ala
        115

<210>  4
<211>  366
<212>  DNA
<213>  Ambrosia artemisiifolia

<220>
<221>  sig_peptide
<222>  (1)..(72)

<220>
<221>  CDS
<222>  (1)..(366)

<400>  4
atg gct aac aaa agt ttg ggc atc ttc atc ctc ata gca aca cta gta        48
Met Ala Asn Lys Ser Leu Gly Ile Phe Ile Leu Ile Ala Thr Leu Val
1               5                   10                  15

gct caa act aca tgg gtt cga gca gtg gac cat ata gtt gga gag gag        96
Ala Gln Thr Thr Trp Val Arg Ala Val Asp His Ile Val Gly Glu Glu
            20                  25                  30

aaa ggt tgg gtc acg ggt gtc gac ttc gaa gct tgg gca aaa gcg agg       144
Lys Gly Trp Val Thr Gly Val Asp Phe Glu Ala Trp Ala Lys Ala Arg
        35                  40                  45

aca ttc tct gta gga gac caa ctt gtg ttc aaa tat gct ccg aat tat       192
Thr Phe Ser Val Gly Asp Gln Leu Val Phe Lys Tyr Ala Pro Asn Tyr
    50                  55                  60

ggt cta ttg ctt acg gat aaa gtg cgt tat gac ata tgt tgt cct cga       240
Gly Leu Leu Leu Thr Asp Lys Val Arg Tyr Asp Ile Cys Cys Pro Arg
65                  70                  75                  80

gga cca att ttg gca cta gaa tct act gga gat act acg gtc aaa ata       288
Gly Pro Ile Leu Ala Leu Glu Ser Thr Gly Asp Thr Thr Val Lys Ile
                85                  90                  95

caa ggg cca ggc gac tat tac ttt att tcg tac agg cct tat tgt ttt       336
Gln Gly Pro Gly Asp Tyr Tyr Phe Ile Ser Tyr Arg Pro Tyr Cys Phe
            100                 105                 110

gag gga tgt aag ctt cac atc acc gtc aag                                    366
Glu Gly Cys Lys Leu His Ile Thr Val Lys
        115                 120

<210>  5
<211>  122
<212>  PRT
<213>  Ambrosia artemisiifolia

<400>  5

Met Ala Asn Lys Ser Leu Gly Ile Phe Ile Leu Ile Ala Thr Leu Val
1               5                   10                  15

Ala Gln Thr Thr Trp Val Arg Ala Val Asp His Ile Val Gly Glu Glu
            20                  25                  30

Lys Gly Trp Val Thr Gly Val Asp Phe Glu Ala Trp Ala Lys Ala Arg
        35                  40                  45

Thr Phe Ser Val Gly Asp Gln Leu Val Phe Lys Tyr Ala Pro Asn Tyr
    50                  55                  60

Gly Leu Leu Leu Thr Asp Lys Val Arg Tyr Asp Ile Cys Cys Pro Arg
65                  70                  75                  80

Gly Pro Ile Leu Ala Leu Glu Ser Thr Gly Asp Thr Thr Val Lys Ile
                85                  90                  95

Gln Gly Pro Gly Asp Tyr Tyr Phe Ile Ser Tyr Arg Pro Tyr Cys Phe
            100                 105                 110

Glu Gly Cys Lys Leu His Ile Thr Val Lys
        115                 120

<210>  6
<211>  98
<212>  PRT
<213>  Ambrosia artemisiifolia

<400>  6

Val Asp His Ile Val Gly Glu Glu Lys Gly Trp Val Thr Gly Val Asp
1               5                   10                  15

Phe Glu Ala Trp Ala Lys Ala Arg Thr Phe Ser Val Gly Asp Gln Leu
            20                  25                  30

Val Phe Lys Tyr Ala Pro Asn Tyr Gly Leu Leu Leu Thr Asp Lys Val
        35                  40                  45

Arg Tyr Asp Ile Cys Cys Pro Arg Gly Pro Ile Leu Ala Leu Glu Ser
    50                  55                  60

Thr Gly Asp Thr Thr Val Lys Ile Gln Gly Pro Gly Asp Tyr Tyr Phe
65                  70                  75                  80

Ile Ser Tyr Arg Pro Tyr Cys Phe Glu Gly Cys Lys Leu His Ile Thr
                85                  90                  95

Val Lys

<210>  7
<211>  432
<212>  DNA
<213>  Ambrosia artemisiifolia

<220>
<221>  sig_peptide
<222>  (1)..(72)

<220>
<221>  CDS
<222>  (1)..(432)

<400>  7
atg gct aac aaa aat ttg tgc atc ttc gtc ctc ata gca aca ata gtg      48
Met Ala Asn Lys Asn Leu Cys Ile Phe Val Leu Ile Ala Thr Ile Val
1               5                   10                  15

gct caa gct aca tgg gtt cga gct aag gac tat cag gtt gga gat atg      96
Ala Gln Ala Thr Trp Val Arg Ala Lys Asp Tyr Gln Val Gly Asp Met
            20                  25                  30

gga gat ggt aat tat aaa gga tta ggt tgg tgc gag act act gat ttc      144
Gly Asp Gly Asn Tyr Lys Gly Leu Gly Trp Cys Glu Thr Thr Asp Phe
        35                  40                  45

caa gct tgg ctc aag ggg aag gag ttc ttt gag gga gac aga ctt tac      192
Gln Ala Trp Leu Lys Gly Lys Glu Phe Phe Glu Gly Asp Arg Leu Tyr
    50                  55                  60

ttc ggt tac aaa gcg gga gaa cat aat gta gtg atg cta aaa gat aaa      240
Phe Gly Tyr Lys Ala Gly Glu His Asn Val Val Met Leu Lys Asp Lys
65                  70                  75                  80

gcc gcg tta gac aca tgt act ctt act aat cca caa ttt gca acc cct      288
Ala Ala Leu Asp Thr Cys Thr Leu Thr Asn Pro Gln Phe Ala Thr Pro
                85                  90                  95

tct tgt gga tat aat tta gtc act tta ggc gcg gca ccc tat tac atc      336
Ser Cys Gly Tyr Asn Leu Val Thr Leu Gly Ala Ala Pro Tyr Tyr Ile
            100                 105                 110

acc agt ggc atg aat gat gat tgt aag aaa ggc atg aag ttg gaa atc      384
Thr Ser Gly Met Asn Asp Asp Cys Lys Lys Gly Met Lys Leu Glu Ile
        115                 120                 125

aat gcc aag ccg agt cct gca ttg ctg aaa gga aga agc atg aag ctt     432
Asn Ala Lys Pro Ser Pro Ala Leu Leu Lys Gly Arg Ser Met Lys Leu
    130                 135                 140

<210>  8
<211>  144
<212>  PRT
<213>  Ambrosia artemisiifolia

<400>  8

Met Ala Asn Lys Asn Leu Cys Ile Phe Val Leu Ile Ala Thr Ile Val
1               5                   10                  15

Ala Gln Ala Thr Trp Val Arg Ala Lys Asp Tyr Gln Val Gly Asp Met
            20                  25                  30

Gly Asp Gly Asn Tyr Lys Gly Leu Gly Trp Cys Glu Thr Thr Asp Phe
        35                  40                  45

Gln Ala Trp Leu Lys Gly Lys Glu Phe Phe Glu Gly Asp Arg Leu Tyr
    50                  55                  60

Phe Gly Tyr Lys Ala Gly Glu His Asn Val Val Met Leu Lys Asp Lys
65                  70                  75                  80

Ala Ala Leu Asp Thr Cys Thr Leu Thr Asn Pro Gln Phe Ala Thr Pro
                85                  90                  95

Ser Cys Gly Tyr Asn Leu Val Thr Leu Gly Ala Ala Pro Tyr Tyr Ile
            100                 105                 110

Thr Ser Gly Met Asn Asp Asp Cys Lys Lys Gly Met Lys Leu Glu Ile
        115                 120                 125

Asn Ala Lys Pro Ser Pro Ala Leu Leu Lys Gly Arg Ser Met Lys Leu
    130                 135                 140

<210>  9
<211>  120
<212>  PRT
<213>  Ambrosia artemisiifolia

<400>  9

Lys Asp Tyr Gln Val Gly Asp Met Gly Asp Gly Asn Tyr Lys Gly Leu
1               5                   10                  15

Gly Trp Cys Glu Thr Thr Asp Phe Gln Ala Trp Leu Lys Gly Lys Glu
            20                  25                  30

Phe Phe Glu Gly Asp Arg Leu Tyr Phe Gly Tyr Lys Ala Gly Glu His
        35                  40                  45

Asn Val Val Met Leu Lys Asp Lys Ala Ala Leu Asp Thr Cys Thr Leu
    50                  55                  60

Thr Asn Pro Gln Phe Ala Thr Pro Ser Cys Gly Tyr Asn Leu Val Thr
65                  70                  75                  80

Leu Gly Ala Ala Pro Tyr Tyr Ile Thr Ser Gly Met Asn Asp Asp Cys
                85                  90                  95

Lys Lys Gly Met Lys Leu Glu Ile Asn Ala Lys Pro Ser Pro Ala Leu
            100                 105                 110

Leu Lys Gly Arg Ser Met Lys Leu
        115                 120

<210>  10
<211>  366
<212>  DNA
<213>  Ambrosia artemisiifolia

<220>
<221>  sig_peptide
<222>  (1)..(72)

<220>
<221>  CDS
<222>  (1)..(366)

<400>  10
atg gct aac aaa agt ttg ggc atc ttc atc ctg ata gca aca ata gta        48
Met Ala Asn Lys Ser Leu Gly Ile Phe Ile Leu Ile Ala Thr Ile Val
1               5                   10                  15

gct caa gct aca tgg gtt cga gca gtg gac cat ata gtt gga gat gat        96
Ala Gln Ala Thr Trp Val Arg Ala Val Asp His Ile Val Gly Asp Asp
            20                  25                  30

aaa ggt tgg gtc aat aat ttc gac tac gaa gct tgg gcc aaa ggg agg       144
Lys Gly Trp Val Asn Asn Phe Asp Tyr Glu Ala Trp Ala Lys Gly Arg
        35                  40                  45

aca ttc tct gtg gga gac caa ctt gtg ttc aat tac gat ccg aaa tat       192
Thr Phe Ser Val Gly Asp Gln Leu Val Phe Asn Tyr Asp Pro Lys Tyr
    50                  55                  60

ggt cta ttg ctt acg gat aaa gtg cgt tat gac ata tgt tgt cct cgg       240
Gly Leu Leu Leu Thr Asp Lys Val Arg Tyr Asp Ile Cys Cys Pro Arg
65                  70                  75                  80

gga cca att ttg gca ttt gaa tct act gga cat acc gtg gcc aaa ata       288
Gly Pro Ile Leu Ala Phe Glu Ser Thr Gly His Thr Val Ala Lys Ile
                85                  90                  95

caa ggg ccg ggc gac tat tac ttt att tcg tat agg cct tat tgt ttt     336
Gln Gly Pro Gly Asp Tyr Tyr Phe Ile Ser Tyr Arg Pro Tyr Cys Phe
            100                 105                 110

gag aaa tgt aag ctt cac atc act gtc aag                             366
Glu Lys Cys Lys Leu His Ile Thr Val Lys
        115                 120

<210>  11
<211>  122
<212>  PRT
<213>  Ambrosia artemisiifolia

<400>  11

Met Ala Asn Lys Ser Leu Gly Ile Phe Ile Leu Ile Ala Thr Ile Val
1               5                   10                  15

Ala Gln Ala Thr Trp Val Arg Ala Val Asp His Ile Val Gly Asp Asp
            20                  25                  30

Lys Gly Trp Val Asn Asn Phe Asp Tyr Glu Ala Trp Ala Lys Gly Arg
        35                  40                  45

Thr Phe Ser Val Gly Asp Gln Leu Val Phe Asn Tyr Asp Pro Lys Tyr
    50                  55                  60

Gly Leu Leu Leu Thr Asp Lys Val Arg Tyr Asp Ile Cys Cys Pro Arg
65                  70                  75                  80

Gly Pro Ile Leu Ala Phe Glu Ser Thr Gly His Thr Val Ala Lys Ile
                85                  90                  95

Gln Gly Pro Gly Asp Tyr Tyr Phe Ile Ser Tyr Arg Pro Tyr Cys Phe
            100                 105                 110

Glu Lys Cys Lys Leu His Ile Thr Val Lys
        115                 120

<210>  12
<211>  98
<212>  PRT
<213>  Ambrosia artemisiifolia

<400>  12

Val Asp His Ile Val Gly Asp Asp Lys Gly Trp Val Asn Asn Phe Asp
1               5                   10                  15

Tyr Glu Ala Trp Ala Lys Gly Arg Thr Phe Ser Val Gly Asp Gln Leu
            20                  25                  30

Val Phe Asn Tyr Asp Pro Lys Tyr Gly Leu Leu Leu Thr Asp Lys Val
        35                  40                  45

Arg Tyr Asp Ile Cys Cys Pro Arg Gly Pro Ile Leu Ala Phe Glu Ser
    50                  55                  60

Thr Gly His Thr Val Ala Lys Ile Gln Gly Pro Gly Asp Tyr Tyr Phe
65                  70                  75                  80

Ile Ser Tyr Arg Pro Tyr Cys Phe Glu Lys Cys Lys Leu His Ile Thr
                85                  90                  95

Val Lys

<210>  13
<211>  15
<212>  PRT
<213>  Artificial Sequence

<220>
<223>  peptide 35-49 of precursor protein A

<400>  13

Gly Trp Ala Leu Gly Val Asp Tyr Val Ala Trp Ala Asn Ala Arg
1               5                   10                  15

<210>  14
<211>  15
<212>  PRT
<213>  Artificial Sequence

<220>
<223>  peptide 50-64 of precursor protein A

<400>  14

Gln Ile Val Gln Gly Asp Glu Leu Val Phe Asn Tyr Asp Val Lys
1               5                   10                  15

<210>  15
<211>  10
<212>  PRT
<213>  Artificial Sequence

<220>
<223>  peptide 65-74 of pro-protein A

<400>  15

Gly Asp Phe Pro Val Phe Tyr Thr Thr Lys
1               5                   10

<210>  16
<211>  31
<212>  PRT
<213>  Artificial Sequence

<220>
<223>  peptide 80-110 of pro-protein A

<400>  16

Cys Cys Pro Tyr Gly Ala Leu Met Glu Leu Ala Asn Thr Gly His Asn
1               5                   10                  15

Val Val Thr Leu Gly Gly Val Gly Asp Tyr Tyr Phe Ile Pro Lys
            20                  25                  30

<210>  17
<211>  13
<212>  PRT
<213>  Artificial Sequence

<220>
<223>  peptide 34-46 of pro-protein B

<400>  17

Gly Trp Val Thr Gly Val Asp Phe Glu Ala Trp Ala Lys
1               5                   10

<210>  18
<211>  11
<212>  PRT
<213>  Artificial Sequence

<220>
<223>  peptide 49-59 of pro-protein B

<400>  18

Thr Phe Ser Val Gly Asp Gln Leu Val Phe Lys
1               5                   10

<210>  19
<211>  12
<212>  PRT
<213>  Artificial Sequence

<220>
<223>  peptide 60-71 of pro-protein B

<400>  19

Tyr Ala Pro Asn Tyr Gly Leu Leu Leu Thr Asp Lys
1               5                   10

<210>  20
<211>  14
<212>  PRT

<213>  Artificial Sequence

<220>
<223>  peptide 60-73 of pro-protein B

<400>  20

Tyr Ala Pro Asn Tyr Gly Leu Leu Leu Thr Asp Lys Val Arg
1               5                   10


<210>  21
<211>  15
<212>  PRT
<213>  Artificial Sequence

<220>
<223>  peptide 81-95 of pro-protein B

<400>  21

Gly Pro Ile Leu Ala Leu Glu Ser Thr Gly Asp Thr Thr Val Lys
1               5                   10                  15


<210>  22
<211>  21
<212>  PRT
<213>  Artificial Sequence

<220>
<223>  peptide 96-116 of pro-protein B

<400>  22

Ile Gln Gly Pro Gly Asp Tyr Tyr Phe Ile Ser Tyr Arg Pro Tyr Cys
1               5                   10                  15


Phe Glu Gly Cys Lys
            20


<210>  23
<211>  438
<212>  DNA
<213>  Artificial Sequence

<220>
<223>  Sequence cloned in the pET 29 vector to produce the rProtein A
       (HisCter)


<220>
<221>  CDS
<222>  (16)..(417)

<400>  23
gaaggagata tacat atg gtg gat cat att gtt ggc gac gac aaa ggc tgg        51
                 Met Val Asp His Ile Val Gly Asp Asp Lys Gly Trp
                 1               5                   10

gtt aac aac ttc gac tac gaa gcc tgg gca aaa ggt cgt acc ttt agc        99

Val Asn Asn Phe Asp Tyr Glu Ala Trp Ala Lys Gly Arg Thr Phe Ser
        15                  20                  25

gtt ggc gat cag ctg gtc ttc aac tac gac ccg aaa tac ggt ctg ctg        147
Val Gly Asp Gln Leu Val Phe Asn Tyr Asp Pro Lys Tyr Gly Leu Leu
    30                  35                  40

ctg acc gat aaa gtt cgt tac gac att tgc tgt ccg cgc ggt ccg att        195
Leu Thr Asp Lys Val Arg Tyr Asp Ile Cys Cys Pro Arg Gly Pro Ile
45                  50                  55                  60

tta gca ttt gaa agc acc ggt cat acc gtt gca aaa atc cag ggt ccg        243
Leu Ala Phe Glu Ser Thr Gly His Thr Val Ala Lys Ile Gln Gly Pro
                65                  70                  75

ggc gat tac tac ttc att agc tac cgc ccg tac tgc ttc gag aaa tgc        291
Gly Asp Tyr Tyr Phe Ile Ser Tyr Arg Pro Tyr Cys Phe Glu Lys Cys
            80                  85                  90

aaa ctg cac atc acc gtt aaa ggc agc ggt acc ctg gtg cca cgc ggt        339
Lys Leu His Ile Thr Val Lys Gly Ser Gly Thr Leu Val Pro Arg Gly
        95                  100                 105

tcc atg gcg ata tcg gat ccg aat tcg agc tcc gtc gac aag ctt gcg        387
Ser Met Ala Ile Ser Asp Pro Asn Ser Ser Ser Val Asp Lys Leu Ala
    110                 115                 120

gcc gca ctc gag cac cac cac cac cac cac tgagatccgg ctgctaacaa a        438
Ala Ala Leu Glu His His His His His His
125                 130


<210>  24
<211>  134
<212>  PRT
<213>  Artificial Sequence

<220>
<223>  Synthetic Construct

<400>  24

Met Val Asp His Ile Val Gly Asp Asp Lys Gly Trp Val Asn Asn Phe
1               5                   10                  15


Asp Tyr Glu Ala Trp Ala Lys Gly Arg Thr Phe Ser Val Gly Asp Gln
            20                  25                  30


Leu Val Phe Asn Tyr Asp Pro Lys Tyr Gly Leu Leu Leu Thr Asp Lys
        35                  40                  45


Val Arg Tyr Asp Ile Cys Cys Pro Arg Gly Pro Ile Leu Ala Phe Glu
    50                  55                  60


Ser Thr Gly His Thr Val Ala Lys Ile Gln Gly Pro Gly Asp Tyr Tyr
65                  70                  75                  80


Phe Ile Ser Tyr Arg Pro Tyr Cys Phe Glu Lys Cys Lys Leu His Ile

85                  90                  95

Thr Val Lys Gly Ser Gly Thr Leu Val Pro Arg Gly Ser Met Ala Ile
            100                 105                 110

Ser Asp Pro Asn Ser Ser Ser Val Asp Lys Leu Ala Ala Ala Leu Glu
        115                 120                 125

His His His His His His
    130

<210>  25
<211>  732
<212>  DNA
<213>  Artificial Sequence

<220>
<223>  Sequence cloned in the pET 28 vector to produce the rProtein A
       (trx)

<400>  25
atgggcagcc accatcacca tcatcactct ggtatgagcg acaagatcat ccacctgacc      60

gacgattcct tcgacaccga tgtcctgaag gcagatggtg caatcctggt ggatttctgg     120

gcagagtggt gtggtccttg taagatgatc gctccaattc tggacgagat cgcagacgag     180

taccaaggta aactgaccgt ggcaaagctg aacatcgacc agaacccagg taccgctccg     240

aaatacggta tccgtggtat cccgactctg ctgctgttta aaaacggtga agtcgcggct     300

actaaagtgg gcgcactgtc taaaggtcag ctgaaagaat tcctggacgc gaacctggct     360

ctggaagtgc tgttccaagg tccggctact cagcacattg ttggtgacga caaaggttgg     420

gctctgggcg ttgattacgt agcgtgggcc aatgcccgtc agattgttca gggcgatgaa     480

ctggttttta actacgatgt taaaggcgac ttcccggttt tctacacgac caaagacaaa     540

ttcgaacgct gctgcccgta tggcgcgctg atggaactgg cgaacaccgg ccacaacgta     600

gttacgctgg gcggcgttgg cgattattat tttatcccga aaggcgtaga ttgcaaacag     660

aacatgaaac tgcacgtgaa cgtaaaagcg tccccggcgc tgcaggaagg ccgtaatgcc     720

attctggcct aa                                                         732

<210>  26
<211>  243
<212>  PRT
<213>  Artificial Sequence

<220>
<223>  rProtein A (trx).

<400>  26

Met Gly Ser His His His His His His Ser Gly Met Ser Asp Lys Ile

1               5                   10                  15

Ile His Leu Thr Asp Asp Ser Phe Asp Thr Asp Val Leu Lys Ala Asp
            20                  25                  30

Gly Ala Ile Leu Val Asp Phe Trp Ala Glu Trp Cys Gly Pro Cys Lys
        35                  40                  45

Met Ile Ala Pro Ile Leu Asp Glu Ile Ala Asp Glu Tyr Gln Gly Lys
    50                  55                  60

Leu Thr Val Ala Lys Leu Asn Ile Asp Gln Asn Pro Gly Thr Ala Pro
65                  70                  75                  80

Lys Tyr Gly Ile Arg Gly Ile Pro Thr Leu Leu Leu Phe Lys Asn Gly
                85                  90                  95

Glu Val Ala Ala Thr Lys Val Gly Ala Leu Ser Lys Gly Gln Leu Lys
            100                 105                 110

Glu Phe Leu Asp Ala Asn Leu Ala Leu Glu Val Leu Phe Gln Gly Pro
        115                 120                 125

Ala Thr Gln His Ile Val Gly Asp Asp Lys Gly Trp Ala Leu Gly Val
    130                 135                 140

Asp Tyr Val Ala Trp Ala Asn Ala Arg Gln Ile Val Gln Gly Asp Glu
145                 150                 155                 160

Leu Val Phe Asn Tyr Asp Val Lys Gly Asp Phe Pro Val Phe Tyr Thr
                165                 170                 175

Thr Lys Asp Lys Phe Glu Arg Cys Cys Pro Tyr Gly Ala Leu Met Glu
            180                 185                 190

Leu Ala Asn Thr Gly His Asn Val Val Thr Leu Gly Gly Val Gly Asp
        195                 200                 205

Tyr Tyr Phe Ile Pro Lys Gly Val Asp Cys Lys Gln Asn Met Lys Leu
    210                 215                 220

His Val Asn Val Lys Ala Ser Pro Ala Leu Gln Glu Gly Arg Asn Ala
225                 230                 235                 240

Ile Leu Ala